# EUROPÄISCHE PATENTSCHRIFT

(11) **EP 0 739 988 B1**
(45) Veröffentlichungstag und Bekanntmachung des Hinweises auf die Patenterteilung: **06.11.2002**
(21) Anmeldenummer: 96106728.7
(22) Anmeldetag: 29.04.1996
(51) Int. Cl.: C12Q 1/68, C12P 19/34, C07H 21/04

(54) **Verfahren zur Gattungs- und speziesspezifische Identifizierung von Legionellen**
Method for genus and species specific identification of Legionella
Procédé pour l'identification du genre et spécifique de espèce de Legionella

(30) Priorität: 29.04.1995 DE 19515891
(43) Veröffentlichungstag der Anmeldung: 30.10.1996
(73) Patentinhaber: Roche Diagnostics GmbH, 68298 Mannheim (DE)
(72) Erfinder: Heidrich, Björn, 10783 Berlin (DE); Robinson, Peter-Nicholas, Dr., 10629 Berlin (DE); Tiecke, Frank, 13057 Berlin (DE); Rolfs, Arndt, Dr., 10999 Berlin (DE)

(56) Entgegenhaltungen:
- WO-A-92/11273
- WO-A-94/28174
- MOLECULAR AND CELLULAR PROBES, Bd. 8, Nr. 1, Februar 1994, Seiten 11-14, XP000578308 MAIWALD M ET AL: "Characterization of contaminating DNA in Taq polymerase which occurs during amplification with a primer set for Legionella 5S ribosomal RNA"
- J.CLIN. MICROBIOL, Bd. 32, Nr. 6, Juni 1994, Seiten 1503-5, XP000579096 MATSIOTA-BERNARD P ET AL: "Evaluation of commercial amplification kit for detection of Legionella pneumophila in clinical specimens"
- CANADIAN JOURNAL OF MICROBIOL., Bd. 40, Nr. 6, Juni 1994, Seiten 495-9, XP000579140 OSHIRO R ET AL: "modification of reagents in the EnviroAmpTm kit to increase recovery of Legionella organisms in water "
- J. CLIN. MICROBIOL., Bd. 31, Nr. 12, Dezember 1993, Seiten 3325-28, XP000579097 KESSLER, H. ET AL.: "Rapid detection of Legionella species in Bronchoaveolar lavage fluids with the EnviroAmp Leginella PCR amplification and detection kit"
- KLI. LABOR., Bd. 40, Nr. 3, 1994, Seiten 211-16, XP000579246 HEIDRICH B ET AL: "Genetische Verwandtschaft innerhalb des Genus Legionalla DNS Sequenzuntersuchung an ribosomalen Genen"
- EUR. J. MICROBIOL. INFECT. DIS.I, Bd. 13, Nr. 3, März 1994, Seiten 225-31, XP000579208 LISBY G ET AL: "Construction of a DNA amplification assay for the detection of Leginella species in clinical samples"
- MED MICROBIOL LETT, Bd. 3, Nr. 6, 1994, Seiten 279-90, XP000579098 HEIDRICH B ET AL: "Automated direct sequencing of Leginella 5S RDNA"

### SEQUENZPROTOKOLL

(1) ALLGEMEINE ANGABEN:
   (i) ANMELDER:
      (A) NAME: Boehringer Mannheim GmbH
      (B) STRASSE: Sandhoferstr.116
      (C) ORT: Mannheim
      (E) LAND: DE
      (F) POSTLEITZAHL: 68305
      (G) TELEFON: 0621 759 4348
      (H) TELEFAX: 0621 759 4457
   (ii) BEZEICHNUNG DER ERFINDUNG: Gattungs- und speziesspezifische Identifizierung von Legionellen
   (iii) ANZAHL DER SEQUENZEN: 68
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Konsensussequenz"
   (vi) URSPRÜNGLICHE HERKUNFT:
      (A) ORGANISMUS: Legionella
   (xi) SEQUENZBESCHREIBUNG: SEQ ID NO: 1:
   GCCTCCCTCA AGATGAGTTT TCCCATGAAG CCCGTTGAAG ACTACGACGT TGATAGGCAA     60
   GGTGTGGAAG CGCAGTAATG GGTGAAGCTA ACTTGTACTA ATTGGCTGAT TGTCTTGACC    120
   ATATAATCTG AGTGACTTCA GAATGTGATA TTGATTTGTA TACGTGAAAC GTATCGTGTA    180
   AACTCTGACT CTTTAGCAAA CCTGTGGCTT AATATAGCAA TCAAAGCCTC AGGTAAACCA    240
   GTTTTCCTGG CGACTATAGC GATTTGGAAC CACCTGATAC CATCTGGAAC TCAGAAGTGA    300
   AACATTTCCG CGCCAATGAT AGTGTGAGGC TTCCTC                              336
(2) ANGABEN ZU SEQ ID NO: 2:
   (i) SEQUENZKENNZEICHEN:
      (A) LANGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 2:
   GGCTGATTGT CTTGACCA                                                   18
(2) ANGABEN ZU SEQ ID NO: 3:
   (i) SEQUENZKENNZEICHEN:
      (A) LANGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 3:
   AGGAAGCCTC ACACTATCAT 20
(2) ANGABEN ZU SEQ ID NO: 4:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 4:
   GTTGAAGACT ACGACGTTGA TAGG 24
(2) ANGABEN ZU SEQ ID NO: 5:
   (i) SEQUENZKENNZEICHEN:
      (A) LANGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desC = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 5:
   AATGTTTCAC TTCTGAGTTC G 21
(2) ANGABEN ZU SEQ ID NO: 6:
   (i) SEQUENZKENNZEICHEN:
      (A) LANGE: 29 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 6:
   AACCACCTGA TACCATCTCG AACTCAGAA 29
(2) ANGABEN ZU SEQ ID NO: 7:
   (i) SEQUENZKENNZEICHEN:
      (A) LANGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      A) ORGANISMUS: Legionella pneumophila
   (xi) SEQUENZBESCHREIBUNG: SEQ ID NO: 7:
   ACGTGAAACG TATCGTGTAA ACTCTGACTC 30
(2) ANGABEN ZU SEQ ID NO: 8:
   (i) SEQUENZKENNZEICHEN:
      (A) LÄNGE: 26 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella anisa
   (xi) SEQUENZBESCHREIBUNG: SEQ ID NO: 8:
   ATGCGAATAC AAGATGTAGG TTGGGC 26
(2) ANGABEN ZU SEQ ID NO: 9:
   (i) SEQUENZKENNZEICHEN:
      (A) LANGE: 34 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella micdadei
   (xi) SEQUENZBESCHREIBUNG: SEQ ID NO: 9:
   ATGTAAATTG CTCAGACAAA TGAATACAGA GTTT 34
(2) ANGABEN ZU SEQ ID NO: 10:
   (i) SEQUENZKENNZEICHEN:
      (A) LANGE: 31 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella brunensis
   (xi) SEQUENZBESCHREIBUNG: SEQ ID NO: 10:
   CCTGTTTTTA CAGAGCACTT AACAATGCTC T                                31
(2) ANGABEN ZU SEQ ID NO: 11:
   (i) SEQUENZKENNZEICHEN:
      (A) LANGE: 29 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella cherrii
   (xi) SEQUENZBESCHREIBUNG: SEQ ID NO: 11:
   AATGCAAATA CAAGAAATTT AGGTTGGGC                                   29
(2) ANGABEN ZU SEQ ID NO: 12:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT;
      (A) ORGANISMUS: Legionella cincinnatensis
   (xi) SEQUENZBESCHREIBUNG: SEQ ID NO: 12:
   CTCTCTTTRT TTACCGGAAG TAACGCG 27
(2) ANGABEN ZU SEQ ID NO: 13:
   (i) SEQUENZKENNZEICHEN:
      (A) LANGE: 26 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella dumoffii
   (xi) SEQUENZBESCHREIBUNG: SEQ ID NO: 13:
   ATCAATACCT GGGGTAGGAC ACCTGC 26
(2) ANGABEN ZU SEQ ID NO: 14:
   (i) SEQUENZKENNZEICHEN:
      (A) LANGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonudeotide"
   (iii) HYPOTHETISCH: NEIN
   (iv) ANTISENSE: JA
   (vi) URSPRÜNGLICHE HERKUNFT:
      (A) ORGANISMUS: Legionella erythra
   (xi) SEQUENZBESCHREIBUNG: SEQ ID NO: 14:
   AACCCGGGTA AGACCGGAAA AACC 24
(2) ANGABEN ZU SEQ ID NO: 15:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (Vi) URSPRÜNGLICHE HERKUNFT:
      (A) ORGANISMUS: Legionella feeleii
   (xi) SEQUENZBESCHREIBUNG: SEQ ID NO: 15:
   GCAAAAATGA AAGACAAATG CGTTTGT 27
(2) ANGABEN ZU SEQ ID NO: 16:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS Legionella israelensis
   (xi) SEQUENZBESCHREIBUNG: SEQ ID NO: 16:
   TTAAACGCTT GTGAATCAAA CCCATTC 27
(2) ANGABEN ZU SEQ ID NO: 17:
   (i) SEQUENZKENNZEICHEN:
      (A) LÄNGE: 27 Basenpaare
      (B) ART: Nucleotid
      (C) STRANGFORM: Einzelatrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella jordanis
   (xi) SEQUENZBESCHREIBUNG: SEQ ID NO: 17:
   TGATGAATGA ATATCCCCTA ACATGGG 27
(2) ANGABEN ZU SEQ ID NO: 18:
   (i) SEQUENZKENNZEICHEN:
      (A) LANGE: 39 Basenpaare
      (B) ART: Nucleotid
      (C) STRANGFORM: Einzelatrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella longbeachae
   (xi) SEQUENZBESCHREIBUNG: SEQ ID NO: 18:
   TGCTTGATAT AAGATATAAT ACCTCTTTAT TTACCTGAG 39
(2) ANGABEN ZU SEQ ID NO: 19:
   (i) SEQUENZKENNZEICHEN:
      (A) LANGE: 32 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella maceachernii
   (xi) SEQUENZBESCHREIBUNG: SEQ ID NO: 19:
   GGCAATACTT TAATTAAAGG CATTAATGCC TA                    32
(2) ANGABEN ZU SEQ ID NO: 20:
   (i) SEQUENZKENNZEICHEN:
      (A) LÄNGE: 23 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella moravica
   (xi) SEQUENZBESCHREIBUNG: SEQ ID NO: 20:
   AGGCCTTGGG CTTGTTGATT GAA                              23
(2) ANGABEN ZU SEQ ID NO: 21:
   (i) SEQUENZKENNZEICHEN:
      (A) LANGE: 40 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella sainthelensis
   (xi) SEQUENZBESCHREIBUNG: SEQ ID NO: 21:
   GTGCTGAATA TAAGATATAA TGTTACTCTC TTTATTTACC 40
(2) ANGABEN ZU SEQ ID NO: 22:
   (i) SEQUENZKENNZEICHEN:
      (A) LANGE: 25 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella spiritensis
   (xi) SEQUENZBESCHREIBUNG: SEQ ID NO: 22:
   GTGTGCCCTG AAGAAGAAAC AGGGT 25
(2) ANGABEN ZU SEQ ID NO: 23:
   (i) SEQUENZKENNZEICHEN:
      (A) LÄNGE: 28 Basenpaare
      (B) ART: Nucleotid
      (C) "STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella steigerwaltii
   (xi) SEQUENZBESCHREIBUNG: SEQ ID NO: 23:
   AATGTGTATA CAAGCTGTAG GTTGGCCA 28
(2) ANGABEN ZU SEQ ID NO: 24:
   (i) SEQUENZKENNZEICHEN:
      (A) LANGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella wadsworthii
   (xi) SEQUENZBESCHREIBUNG: SEQ ID NO: 24:
   GTACGTACGA ATTAGAGATT GGGTCTAGGC 30
(2) ANGABEN ZU SEQ ID NO: 25:
   (i) SEQUENZKENNZEICHEN:
      (A) LANGE: 31 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "DNA-Oligonucleotide"
   (iii) HYPOTHETISCH: NEIN
   (iv) ANTISENSE: JA
   (vi) URSPRÜNGLICHE HERKUNFT:
      (A) ORGANISMUS: Legionella
   (xi) SEQUENZBESCHREIBUNG: SEQ ID NO: 25:
   TTGTAGTAAT TGGCTGATTG TCTTGACCAT A 31
(2) ANGABEN ZU SEQ ID NO: 26:
   (1) SEQUENZKENNZEICHEN:
      (A) LANGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Philadelphia-1
      (C) INDIVIDUUM/ISOLAT: 01phila
   (xi) SEQUENZBESCHREIBUNG: SEQ ID NO: 26:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTAACTTCA GAATRTGATA TTGATTTGTA TACCTGATAM GTATCGTGTA   180
   AACTCTGACT CTTTACCAAA CCTGTGGCTT AATAAAGCAA TCAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 27:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Knoxville-1
      (C) INDIVIDUUM/ISOLAT: 02knox
   (xi) SEQUENZBESCHREIBUNG: SEQ ID NO: 27:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC 120
   ATATAATCTG AGTGACTTCA GAATGTGATA TTGATTTGTA TACGTGAAAC GTATCGTGTA 180
   AACTCTGACT CTTTACCAAA CCTGTGGCTT AATATAGCAA TCAAAGCCTC AGGTAAACCA 240
   GTTTTCCTGG CGACTATAGC GATTTGGAAC CACCTGATAC CATCTCGAAC TCAGAAGTGA 300
   AACATTTCCG CGCCAATGAT AGTGTGAGGC TTCCTC 336
(2) ANGABEN ZU SEQ ID NO: 28:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Benidorm 030E
      (C) INDIVIDUUM/ISOLAT: 04beni
   (xi) SEQUENZBESCHREIBUNG: SEQ ID NO: 28:
   GCCTCCCTCA AGATGAGTTT TCCCATGAAG CCCGTTGAAG ACTACGACGT TGATAGGCAA 60
   GGTGTGGAAG CGCAGTAATG CGTGAAGCTA ACTTGTACTA ATTGGCTGAT TGTCTTGACC 120
   ATATAATCTG AGTGACTTCA GAATGTGATA TTGATTTGTA TACGTGAAAC GTATCGTGTA 180
   AACTCTGACT CTTTACCAAA CCTGTGGCTT AATAATGCAA TCAAAGCCTC AGGTAAMCCA 240
   GTTTTCCTGG CGACTATAGC GATTTGGAAC CACCTGATAC CATCTCGAAC TCAGAAGTGA 300
   AACATTTCCG CGCCAATGAT AGTGTGAGGC TTCCTC 336
(2) ANGABEN ZU SEQ ID NO: 29:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: France 5811
      (C) INDIVIDUUM/ISOLAT: 05fran
   (xi) SEQUENZBESCHREIBUNG: SEQ ID NO: 29:
   GCCTCCCTCA AGATGAGTTT TCCCATGAAG CCCGTTGAAG ACTAGGACGT TGATAGGCAA    60
   GGTGTGGAAG CGCAGTAATG CGTGAAGCTA ACTTGTACTA ATTGGCTGAT TGTCTTGACC   120
   ATATAATCTG AGTRACTTCA GAATGTGATA TTGATTTGTA TACCTGAWAC GTATCGTGTA   180
   AACTCTGACT CTTTACCAAM CCTGTGGCTT AATAWAGCAA TYAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 30:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: OLDA
      (C) INDIVIDUUM/ISOLAT: 06olda
   (xi) SEQUENZBESCHREIBUNG: SEQ ID NO: 30:
   GCCTCCCTCA AGATGAGTTT TCCCATGNNN NNCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GATTATGATA TTGATTTGTA TACGTGAAAC GTATCGTGTA   180
   AACTCTGACT CTTTACCAAA CCTGTGGCTT AACAYAGCAA TCAAAGCCTC AGGTAATCCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 31:
   (i) SEQUENZKENNZEICHEN:
      (A) LÄNGE: 335 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH; NEIN
   (vi) URSPRÜNGLICHE HERKUNFT:
      (A) ORGANISMUS: Legionella pneumophila
      (B) STAMM: Oxford 4032E
      (C) INDIVIDUUM/ISOLAT: 07oxfo
   (xi) SEQUENZBESCHREIBUNG: SEQ ID NO: 31:
   GCCTCCCTCA AGATGAGTTT TCCCATGANN NNCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GAATGTGATT TTGATTTGTA TACGTGAAAC GTATCGTGTA   180
   AACTCTGACT CTTTACCAAA CSTGTGGCTT AATAATGCAA TCAAGCCTCA GGTAAACCAG   240
   TTTTCCTGGC GACTATAGCG ATTTGGAACC ACCTGATACC ATCTCGAACT CAGAAGTGAA   300
   ACATTTCCGC GCCAATGATA GTGTGAGGCT TCCTC                              335
(2) ANGABEN ZU SEQ ID NO: 32:
   (i) SEQUENZKENNZEICHEN:
      (A) LÄNGE: 336 Basenpaare
      (B) ART: Nucleotid
      (C) STRANGFORM: Einzeletrang
      (D) TOPOLOGIE: linear
   (ii) ART DES MOLEKÜLS: Genom-DNA
   (iii) HYPOTHETISCH: NEIN
   (Vi) URSPRÜNGLICHE HERKUNFT:
      (A) ORGANISMUS: Legionella pneumophila
      (B) STAMM: Camperdown-1
      (C) INDIVIDUUM/ISOLAT: 08Camp
   (xi) SEQUENZBESCHREIBUNG: SEQ ID NO: 32:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GATTATGATA TTGATTTGTA TACGTGAAAC GTATCGTGTA   180
   AACTCTGACT CTTTACCAAA CCTGTGGCTT AACATAGCAA TCAAAGCCTC AGGTAATCCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 33:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH; NEIN
   (Vi) URSPRÜNGLICHE HERKUNFT:
      (A) ORGANISMUS: Legionella pneumophila
      (B) STAMM: Togus-1
      (C) INDIVIDUUM/ISOLAT: 09tog
   (xi) SEQUENZBESCHREIBUNG: SEQ ID NO: 33:
   GCCTCCCTCA AGATGAGTTT TCCCATGAAG CCCGTTGAAG ACTACGACGT TGATAGGCAA     60
   GGTGTGGAAG CGCAGTAATG CGTGAAGCTA ACTTGTACTA ATTGGCTGAT TGTCTTGACC    120
   ATATAATCTG AGTGACTTCA GAATGTGATA TTGATTTGTA TACCTGATAA GTATCGTGTA    180
   AACTCTGACT CTTTACCAAA CCTGTGGCTT AATAAAGCAA TMAAAGCCTC AGGTAAMCCA    240
   GTTTTCCTGG CGACTATAGC GATTTGGAAC CACCTGATAC CATCTCGAAC TCAGAAGTGA    300
   AACATTTCCG CGCCAATGAT AGTGTGAGGC TTCCTC                              336
(2) ANGABEN ZU SEQ ID NO: 34:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Bloomington-2
      (C) INDIVIDUUM/ISOLAT: 10Bloom
   (xi) SEQUENZBESCHREIBUNG: SEQ ID NO: 34:
   GCCTCCCTCA AGATGAGTTT TCCCATGANN NCCGTTGAAG ACTACGACGT TGATAGGCAA     60
   GGTGTGGAAG CGCAGTAATG CGTGAAGCTA ACTTGTACTA ATTGGCTGAT TGTCTTGACC    120
   ATATAATCTG AGTGACTTCA GAATGTGATA TTGATTTGTA TACGTGAAAC GTATGGTGTA    180
   AACTCTGACT CTTTACCAAA CCTGTGGCTT AATATAGCAA TCAAAGCCTC AGGTAAACCA    240
   GTTTTCCTGG CGACTATAGC GATTTGGAAC CACCTGATAC CATCTCGAAC TCAGAAGTGA    300
   AACATTTCCG CGCCAATGAT AGTGTGAGGC TTCCTC                              336
(2) ANGABEN ZU SEQ ID NO: 35:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Los Angeles-1
      (C) INDIVIDUUM/ISOLAT: 1lsg41a
   (xi) SEQUENZBESCHREIBUNG: SEQ ID NO: 35:
   GCCTCCCTCA AGATGAGTTT TCCCATGNNN NNCGTTGAAG ACTACGAGGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GAATGTATAA TTGAATTGAA TACGTACAAC GCATCGTGTA   180
   AACTCCGACT CTTTACCAAA CCTGTGGCTT AATAGTGTAA TCAAAGCCTC AGGTAAAGCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 36:
   (i) SEQUENZKENKZEICHEN;
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Portland
      (C) INDIVIDUUM/ISOLAT: 12sg4po
   (xi) SEQUENZBESCHREIBUNG: SEQ ID NO: 36:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTAACTTCA GAATRTGATA TTGATTTGTA TACCTGATAM GTATCGTGTA   180
   AACTCTGACT CTTTACCAAA CCTGTGGCTT AATAAAGCAA TCAAAGCCTC AGGTAAAGCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 37:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETZSCH: NEIN
   (vi) URSPRÜNGLICHE HERKUNFT:
      (A) ORGANISMUS: Legionella pneumophila
      (B) STAMM: Dallas-1E
      (C) INDIVIDUUM/ISOLAT: 13sg5da
   (xi) SEQUENZBESCHREIBUNG: SEQ ID NO: 37:
   GCCTCCCTCA AGATGAGTTT TCCCATGAAG CCCGTTGAAG ACTACGACGT TGATAGGCAA    60
   GGTGTGGAAG CGCAGTAATG YGTGAAGCTA ACTTGTACTA ATTGGCTGAT TGTCTTGACC   120
   ATATAATCTG AGTGACTTCA GAATGTATAA TTGAATTGAA TACGTACAAC GCATCGTGTA   180
   AACTCCGACT CTTTACCAAA CCTGTGGCTT AATAGTGTAA TCAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 38:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Cambridge-2
      (C) INDIVIDUUM/ISOLAT: 14sg5cam
   (xi) SEQUENZBESCHREIBUNG: SEQ ID NO: 38:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC      120
   ATATAATCTG AGTGACTTCA GAATGTGATA TTGATTTGTA TACGTGAAAC GTATCGTGTA      180
   AACTCTGACT CTTTACCAAA CCTGTGGCTT AATACAGCAA TCAAAGCCTC AGGTAAACCA      240
   GTTTTCCTGG CGACTATAGC GATTTGGAAC CACCTGATAC CATCTCGAAC TCAGAAGTGA      300
   AACATTTCCG CGCCAATGAT AGTGTGAGGC TTCCTC                                336
(2) ANGABEN ZU SEQ ID NO: 39:
   (i) SEQUENZKENNZEICHEN:
      (A) LÄNGE: 336 Basenpaare
      (B) ART: Nucleotid
      (C) STRANGFORM; Einzelatrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Chicago-2
      (C) INDIVIDUUM/ISOLAT: 15sg6ch
   (xi) SEQUENZBESCHREIBUNG: SEQ ID NO: 39:
   GCCTCCCTCA AGATGAGTTT TCCCATGANN NNCGTTGAAG ACTACGACGT TGATAGGCAA       60
   GGTGTGGAAG CGCAGTAATG CGTGAAGCTA ACTTGTACTA ATTGGCTGAT TGTCTTGACC      120
   ATATAATCTG AGTAACTTCA GAATATGATA TTGATTTGTA TACCTGATAM GTATGGTGTA      180
   AACTCTGACT CTTTACCAAA CCTGTGGCTT AATAAAGCAA TCAAAGCCTC AGGTAAACCA      240
   GTTTTCCTGG CGACTATAGC GATTTGGAAC CACCTGATAC CATCTCGAAC TCAGAAGTGA      300
   AACATTTCCG CGCCAATGAT AGTGTGAGGC TTCCTC                                336
(2) ANGABEN ZU SEQ ID NO: 40:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Chicago-8
      (C) INDIVIDUUM/ISOLAT: 16sg7
   (xi) SEQUENZBESCHREIBUNG: SEQ ID NO: 40:
   GCCTCCCTCA AGATGAGTTT TCCCATGAAG CCCGTTGAAG ACTACGACGT TGATAGGCAA   60
   GGTGTGGAAG CGCAGTAATG CGTGAAGCTA ACTTGTACTA ATTGGCTGAT TGTCTTGACC  120
   ATATAATCTG AGTGACTTCA GAATGTGATA TTGATTTGTA TACGTGAAAC GTATCGTGTA  180
   AACTCTGACT CTTTACCAAA CCTGTGGCTT AATATAGCAA TCAAAGCCTC AGGTAAACCA  240
   GTTTTCCTGG CGACTATAGC GATTTGGAAC CACCTGATAC CATCTCGAAC TCAGAAGTGA  300
   AACATTTCCG CGCCAATGAT AGTGTGAGGA CTCCTC                            336
(2) ANGABEN ZU SEQ ID NO: 41:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Concord-3
      (C) INDIVIDUUM/ISOLAT: 17sg8
   (xi) SEQUENZBESCHREIBUNG: SEQ ID NO: 41:
   GCCTCCCTCA AGATGAGTTT TCCCATGAAG CCCGTTGAAG ACTACGACGT TGATAGGCAA   60
   GGTGTGGAAG CGCAGTAATG CGTGAAGCTA ACTTGTACTA ATTGGCTGAT TGTCTTGACC  120
   ATATAATCTG AGTGACTTCA GAATGTGATA TTGATTTGTA TACGTGAAAC GTATCGTGTA  180
   AACTCTGACT CTTTACCAAA CCTGTGGCTT AATAAAGCAA GAAAAGCCTC AGGTAAACCA  240
   GTTTTCCTGG CGACTATAGC GATTTGGAAC CACCTGATAC CATCTCGAAC TCAGAAGTGA  300
   AACATTTCCG CGCCAATGAT AGTGTGAGGT CTCCTC                            336
(2) ANGABEN ZU SEQ ID NO: 42:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: IN-23-G1-C2
      (C) INDIVIDUUM/ISOLAT; 18sg9
   (xi) SEQUENZBESCHREIBUNG: SEQ ID NO: 42:
   GCCTCCCTCA AGATGAGTTT TCCCATGANN NCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GATTATGATA TTGATTTGTA TACGTGAAAC GTATCGTGTA   180
   AACTCTGACT CTTTACCAAA CCTGTGGCTT AACATAGCAA TCAAAGCCTC AGGTAATCCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 43:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: Leiden-1
      (C) INDIVIDUUM/ISOLAT: 19sg10
   (xi) SEQUENZBESCHREIBUNG: SEQ ID NO: 43:
   GCCTCCCTCA AGATGAGTTT TCCCATGNNN NNCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GAATGTGATA TTGATTTGTA TACGTGAAAC GTATCGTGTA   180
   AACTCTGACT CTTTACCAAA CCTGTGGCTT AAYAYAGCAA TCAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 44:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: 797-PA-H
      (C) INDIVIDUUM/ISOLAT: 20sg11
   (xi) SEQUENZBESCHREIBUNG: SEQ ID NO: 44:
   GCCTCCCTCA AGATGAGTTT TCCCATGAAG CCCGTTGAAG ACTACGACGT TGATAGGCAA     60
   GGTGTGGAAG CGCAGTAATG CGTGAAGCTA ACTTGTACTA ATTGGCTGAT TGTCTTGACC    120
   ATATAATCTG AGTGACTTCA GAATGTGATA TTGATTTGTA TACGTGAAAC GTATGGTGTA    180
   AACTCTGACT CTTTACCAAA CCTGTGGCTT AATAATGCAA TCAAAGCCTC AGGTAAMCCA    240
   GTTTTCCTGG CGMCTATAGC GATTTGGAAC CACCTGATAC CATCTCGAAC TCAGAAGTGA    300
   AACATTTCCG CGCCAATGAT AGTGTGAGGC TTCCTC                              336
(2) ANGABEN ZU SEQ ID NO: 45:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: 570-CO-H
      (C) INDIVIDUUM/ISOLAT: 21sg12
   (xi) SEQUENZBESCHREIBUNG: SEQ ID NO: 45:
   GCCTCCCTCA AGATGAGTTT TCCCATGNNN NNCGNNGAAG ACTACGACGT TGATAGGCAA     60
   GGTGTGGAAG CGCAGTAATG CGTGAAGCTA ACTTGTACTA ATTGGCTGAT TGTCTTGACC    120
   ATATAATCTG AGTAACTTCA GAATRTGATA TTGATTTGTA TACCTGATAM GTATCGTGTA    180
   AACTCTGACT CTTTACCAAA CCTGTGGCTT AATAAAGCAA TCAAAGCCTC AGGTAAACCA    240
   GTTTTCCTGG CGACTATAGC GATTTGGAAC CACCTGATAC CATCTCGAAC TCAGAAGTGA    300
   AACATTTCCG CGCCAATGAT AGTGTGAGGC TTCCTC                              336
(2) ANGABEN ZU SEQ ID NO: 46:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS Genorn-DNA
   (iii) HYPOTHETISCH: NEIN
   (vi) URSPRÜNGLICHE HERKUNFT:
      (A) ORGANISMUS: Legionella pneumophila
      (B) STAMM: 82-A-3105
      (C) INDIVIDUUM/ISOLAT: 22sg13
   (xi) SEQUENZBESCHREIBUNG: SEQ ID NO: 46:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GAATGTGATA TTGATTTGTA TACGTGAAAC GTATCGTGTA   180
   AACTCTGACT CTTTACCAAA CCTGTGGCTT AATACAGCAA TCAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ ID NO: 47:
   (i) SEQUENZKENNZEICHEN:
      (A) LÄNGE: 336 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella pneumophila
      (B) STAMM: 1169-MN-H
      (C) INDIVIDUUM/ISOLAT: 23sg14
   (xi) SEQUENZBESCHREIBUNG: SEQ ID NO: 47:
   GCCTCCCTCA AGATGAGTTT TCCCATGAAG CCCGTTGAAG ACTACGACGT TGATAGGCAA    60
   GGTGTGGAAG CGCAGTAATG CGTGAAGCTA ACTTGTACTA ATTGGCTGAT TGTCTTGACC   120
   ATATAATCTG AGTGACTTCA GAATGTGATA TTGATTTGTA TACGTGAAAC GTATCGTGTA   180
   AACTCTGACT CTTTACCAAA CCTGTGGCTT AAYAYAGCAA TCAAAGCCTC AGGTAAACCA   240
   GTTTTCCTGG CGACTATAGC GATTTGGAAC CACCTGATAC CATCTCGAAC TCAGAAGTGA   300
   AACATTTCCG CGCCAATGAT AGTGTGAGGC TTCCTC                             336
(2) ANGABEN ZU SEQ 1 NO: 48:
   (i) SEQUENZKENNZEICHEN:
      (A) LÄNGE: 374 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella anisa
      (B) STAMM: WA-316-C2
      (C) INDIVIDUUM/ISOLAT: 24ani
   (xi) SEQUENZBESCHREIBUNG: SEQ ID NO: 48:
(2) ANGABEN ZU SEQ ID NO: 49:
   (i) SEQUENZXENNZEICHEN:
      (A) LANGE: 350 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella brunensis
   (xi) SEQUENZBESCHREIBUNG: SEQ ID NO: 49:
   GCCTCCCTCA AGATGAGTTT TCCCATGAAG CCCGTTGAAG ACTACGACGT TGATAGGCGA    60
   GGTGTGGAAG CGCAGTAATG TGTGAAGCTA ACTCGTACTA ATTGGCTGAT TGTCTTGACC   120
   ATATAACCTG AATGACTTCG GGTTATGATA GAAGATGATA GATTATGCCG TAAGGCACTT   180
   GTGTTAACCC TTTTTTACTT TACCAGCCTG TTTTTACAGA GCACTTAACA ATGCTCTTTA      240
   TCAACAGGAC AACAGTTTTC CTGGCGACCA TAGCGGTTTG GAACCACCTG ACTCCATCTC      300
   GAACTCAGTA GTGAAACAGA CCAGCGCCGA TGATAGTGTG AGGCTTCCTC                 350
(2) ANGABEN ZU SEQ ID NO: 50:
   (i) SEQUENZKENNZEICHEN:
      (A) LÄNGE: 317 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella cincinnatiensis
      (B) STAMM: 72-OH-H
      (C) INDIVIDUUM/ISOLAT: 29cin
   (xi) SEQUENZBESCHREIBUNG: SEQ ID NO: 50:
   GCCTCCCTCA AGCTGAGTTT TCCCTTGAAG CCCGTTGAAG ACTACGACGT TGATAGGCAA       60
   GGTGTGGAAG CGTAGTAATG CGTGAAGCTA ACTTGTACTA ATTGGCTGAT TGTCTTGACC      120
   ATATAATCTG AGTTACTTCA GAGTGAAACA GAATATAAGT GACACCATGA CTCTCTTTRT      180
   TTACCGGAAG TAACGCGCTC CAAGGCGCGC TACTCAAAAC AGTTTTCCTG GCGACCATAG      240
   CGGTTTGGAA CCACCTGATT CCATCTCGAA CTCAGTAGTG AAACGAACAT GCGCCAATGA      300
   TAGTGTGAGG TTTCCTC                                                     317
(2) ANGABEN ZU SEQ ID NO: 51:
   (i) SEQUENZKENNZEICHEN:
      (A) LÄNGE: 359 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella dumoffii
      (B) STAMM: NY-23
      (C) INDIVIDUUM/ISOLAT: 31DUMO
   (xi) SEQUENZBESCHREIBUNG: SEQ ID NO: 51:
   GCCTCCCTCA AGATGAGTTT TCCCATGAAG CCCGTTGGAG ACTACGACGT TGATAGGCAA    60
   GGTGTGGAAG CGCAGTAATG CGTGCAGCTA ACTTGTACTA ATTGGCTGAT TGTCTTGACC   120
   ATATAATCTG AGTTACTTCA GATGAACTGA ATCAATACCT GGGGTAGGAC ACCTGCCCCG   180
   AAATAAATAC AAAATAGTGT GTCCTCTTTA TTTACCTCGT GCATGATTCG GGTATAATAT   240
   GCCCAATTGA TCATGTCAAA CCAGTTTTCC TGGCGACCAT AGCGGTTTGG AACCACCTGA   300
   ATCCATCTCG AACTCAGAAG TGAAACGAAC ATGCGCCAAT GATAGTGTGA GGCTTCCTC    359
(2) ANGABEN ZU SEQ ID NO: 52:
   (i) SEQUENZKENNZEICHEN:
      (A) LÄNGE: 362 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella cherrii
      (B) STAMM: ORW
      (C) INDIVIDUUM/ISOLAT: 30che
   (xi) SEQUENZBESCHREIBUNG: SEQ ID NO: 52:
   GCCTCCCTCA AGATGAGTTT TCCCATGAAG CCCGTTGAAG ACTACGACGT TGATAGGCAA    60
   GGTGTGGAAG CACAGTAATG TGTGCAGCTA ACTTGTACTA ATTGGCTGAT TGTCTTGACC   120
   ATATAATCTG AATTACTTCA GATTAACTGA ATGCAAATAC AAGAAATTTA GGTTGGGCCA   180
   CGGCCCAATC TGCAAAAAAA ATGTGTACTC TTTATTTACC TAACGCATGA TTCGGGTATA   240
   ATGCGCCCAT TAATCATGTT AAACCAGTTT TCCTGGCGAC CATAGCGGTT TGGAACCACC   300
   TGACTCCATC TCGAACTCAG AAGTGAAACG AACCCGCGCC AATGATAGTG TGAGGTTTCC   360
   TC                                                                  362
(2) ANGABEN ZU SEQ ID NO: 53:
   (i) SEQUENZKENNZEICHEN:
      (A) LÄNGE: 325 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella erythra
      (B) STAMM: SE-32A-C8
      (C) INDIVIDUUM/ISOLAT: 32ERY
   (xi) SEQUENZBESCHREIBUNG; SEQ ID NO: 53:
   GCCTCCCTCA AGATGAGTTT TCCCATGAAG CCCGTTGAAG ACTACGACGT TGATAGGCGA    60
   GGTGTGGAAG CGTAGTAATG CGTGAAGCTA ACTCGTACTA ATTGGCTGAT TGTCTTGACT   120
   ATATAACCTG ATGCGCTTCA GGTTATATGG ATAACATGAA TGTGACTCTA TTTTTTACCG   180
   GCCTCGTGGC CAACCCGGGT AAGACCGGAA AAACCATGAT GCTTAAACCG TTTTCCTGGC   240
   GACCATAGCA GTTTGGAACC ACCTGAATCC ATCTCGAACT CAGAAGTGAA ACAGACTGGC   300
   GCCGATGATA GTGTGAGGCT TCCTC                                         325
(2) ANGABEN ZU SEQ ID NO: 54:
   (i) SEQUENZKENNZEICHEN:
      (A) LÄNGE: 342 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella feeleii
      (B) STAMM: WO-44C
      (C) INDIVIDUUM/ISOLAT: 33feel
   (xi) SEQUENZBESCHREIBUNG: SEQ ID NO: 54:
   GCCTCCCTCA AGATGAGTTT TCCCATGAAG CCCGTTGAAG ACTACGACGT TGATAGGCGA    60
   GGTGTGGAAG CGCAGTAATG CGTGAAGCTA ACTCGTACTA ATTGGCTGAT TGTCTTGACC   120
   ATATAACCTG AATTGCTTTG AGGTTATAGG CAAAAATGAA AGACAAATGC GTTTGTGTTA   180
   CCTCATAATC TTTACCGGCC TGCTGGCTGA GCACTTAACC CTGCTTTATC CAGAACAGGC   240
   AAACCCGTTT TCCTGGCGAC CATAGCGGTT TGGAACCACC TGACTCCATC TCGAACTCAG   300
   AAGTGAAACA AACCCGCGCC GATGATAGTG TGGAGTTTCT CC                      342
(2) ANGABEN ZU SEQ ID NO: 55:
   (i) SEQUENZKENNZEICHEN:
      (A) LÄNGE: 349 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella feeleii
      (B) STAMM: 691-WI-H
      (C) INDIVIDUUM/ISOLAT: 34feel
   (xi) SEQUENZBESCHREIBUNG: SEQ ID NO: 55:
   GCGGGAAGCC TCCCTCAAGA TGAGTTTTCC CATGAAGCCC GTTGAAGACT AGGACGTTGA    60
   TAGGCGAGGT GTGGAAGCGC AGTAATGCGT GAAGCTAACT CGTACTAATT GGCTGATTGT   120
   CTTGACCATA TAACCTGAAT TGCTTTGAGG TTATAGGCAA AAATGAAAGA CAAATGCGTT   180
   TGTGTTACCT CATAATCTTT ACCGGCCTGC TGGCTGAGCA CTTAAACCTG CTTTATCCAG   240
   AACAGGCAAA CCCGTTTTCC TGGCGACCAT AGCGGTTTGG AACCACCTGA CTCCATCTCG   300
   AACTCAGAAG TGAAACAAAC CCGCGCCGAT GATAGTGTGG AGTTTCTCC               349
(2) ANGABEN ZU SEQ ID NO: 56:
   (i) SEQUENZKENNZEICHEN:
      (A) LÄNGE: 321 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella israelensis
      (B) STAMM: Bercovier-4
      (C) INDIVIDUUM/ISOLAT: 36isr
   (xi) SEQUENZBESCHREIBUNG: SEQ ID NO: 56:
   GCCTTCCTCA AGATGAGTTT TCCCTTGAAG CCCGTTGAAG ACGACGACGT TGATAGGCGA    60
   GGTGTGGAAG CGCAGTAATG TGTGAAGCTA ACTCGTACTA ATTGGCTGAT TGTCTTGACC   120
   ATATATCCTG AAATCATTCA GGGCATGATA CAAAATGAGT TTAAACGCTT GTGAATCAAA   180
   CCCATTCAAT CTTTACCTTC TGCCTTCAAT AAGGCAGAAT AACCCGTTTT CCTGGCGACC   240
   ATAGCTGTTT GGTACCACCT GATACCTTTC CGAACTCAGT AGTGAAACAA ACAGGCGCTG   300
   ATGATAGTGT GGGGTCTCCC C                                             321
(2) ANGABEN ZU SEQ ID NO: 57:
   (i) SEQUENZKENNZEICHEN:
      (A) LÄNGE: 348 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella jordanis
      (B) STAMM: BL-540
      (C) INDIVIDUUM/ISOLAT: 38jor
   (xi) SEQUENZBESCHREIBUNG: SEQ ID NO: 57:
   GCCTCCCTCA AGATGAGTTT TCCCATGAAG CCCGTTGAAG ACTACGACGT TGATAGGGGA    60
   GGTGTGGAAG CGCAGTAATG TGTGAAGCTA ACTCGTACTA ATTGGCTGAT TGTCTTGACC   120
   ATATAACCTG AATGGCTTTT ATGTGGCAAG TCAAAGACAA GGCTTGGCAA GCTTGTGTTG   180
   CCCTAATATT TATCTTTACC AGCCTGATGA ATGAATATCC CCTAACATGG GTATTTGCTC   240
   AGCAGGACAA CGTTTTTCCT GGCGACCATA GCGGTTTGGA ACCACCTGAC TCCATCTCGA   300
   ACTCAGAAGT GAAACAGACC AGCGCCGATG ATAGTGTGAG GCTTCCTC                348
(2) ANGABEN ZU SEQ ID NO: 58:
   (i) SEQUENZKENNZEICHEN:
      (A) LÄNGE: 312 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella longbeachae sero.1
      (B) STAMM: Long Beach-4
      (C) INDIVIDUUM/ISOLAT: 39long1
   (xi) SEQUENZBESCHREIBUNG: SEQ ID NO: 58:
   GCCTCCCTCA AGATGAGTTT TCCCTTGAAG CCCGTTGAAG ACTACGACGT TGATAGGCAA    60
   GGTGTGGAAG CGTAGTAATG CGTGAAGCTA ACTTGTACTA ATTGGCTGAT TGTCTTGACC   120
   ATATAATCTG AGTTACTTTA GATTATGCTT GATATAAGAT ATAATACCTC TTTATTTACC   180
   TGAGTATCAT GCCAATAATG CGCGATACTC AAAACAGTTT TCCTGGCGAC TATAGCGGTT   240
   TGGAACCACC TGAATCCATC TCGAACTCAG AAGTGAAACG TACATGCGCC AATGATAGTG   300
   TGAGGCTTCC TC                                                       312
(2) ANGABEN ZU SEQ ID NO: 59:
   (i) SEQUENZKENNZEICHEN:
      (A) LÄNGE: 312 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella lonbeachae sero.2
      (B) STAMM: Tucker-1
      (C) INDIVIDUUM/ISOLAT: 40long2
   (xi) SEQUENZBESCHREIBUNG: SEQ ID NO: 59:
   GCCTCCCTCA AGATGAGTTT TCCCTTGAAG CCCGTTGAAG ACTACGACGT TGATAGGCAA    60
   GGTGTGGAAG CGTAGTAATG CGTGAAGCTA ACTTGTACTA ATTGGCTGAT TGTCTTGACC   120
   ATATAATCTG AGTTACTTTA GATTATGCTT GATATAAGAT ATAATACCTC TTTATTTACC   180
   TGAGTATCAT GCCAATAATG CGCGATACTC AAAACAGTTT TCCTGGCGAC TATAGGGGTT   240
   TGGAACCACC TGAATCCATC TCGAACTCAG AAGTGAAACG AACATGCGCC AATGATAGTG   300
   TGAGGCTTCC TC                                                       312
(2) ANGABEN ZU SEQ ID NO: 60:
   (i) SEQUENZKENNZEICHEN:
      (A) LÄNGE: 354 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella machearchernii
      (B) STAMM: PX-1-G2-E2
      (C) INDIVIDUUM/ISOLAT; 41mac
   (xi) SEQUENZBESCHREIBUNG: SEQ ID NO: 60:
   GCCTCCCTCA AGATGAGTTT TCCCATGAAG CCCGTTGGAG ACTACGACGT TGATAGGCGA    60
   GGTGTGGAAG CACAGTAATG TGTGTAGCTA ACTCGTACTA ATTGGCTGAT TGTCTTGACC   120
   ATATAACCTG AGCTGCTTTT AGGTTGAAGA GTAAGTGATA AGGCAATACT TTAATTAAAG   180
   GCATTAATGC CTAAGCGTTT GTGTTAACCT CTAACCCCTT TACCAAGCTG ATTGGCGAAT   240
   AGGCCAATCG GTAAACCAGT TTTCCTGGCG ACCATAGCGG TTTGGAACCA CCTGAATCCA 300
   TCTCGAACTC AGAAGTGAAA CAGACCTGCG CCAATGATAG TGTGGGGCTT CCCC 354
(2) ANGABEN ZU SEQ ID NO: 61:
   (i) SEQUENZKENNZEICHEN:
      (A) LÄNGE: 374 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella micdadei
      (B) STAMM: Tatlock
      (C) INDIVIDUUM/ISOLAT: 42micd
   (xi) SEQUENZBESCHREIBUNG: SEQ ID NO: 61:
   GAAGCCTCCC TCAAGATGAG TTTTCCCATG AAGCCCGTTG AAGACTACGA CGTTGATAGG 60
   CGAGGTGTGG AAGCACAGTA ATGTGTGTAG CTAACTCGTA CTAATTGGCT GATTGTCTTG 120
   ACCATATAAC CTGAACTGCC TTTAGGTTAT GAGTGAAGAA GCAAGGCAAT ATTGAATGAC 180
   AGGGCAATGT AAATTGCTCA GACAAATGAA TACAGAGTTT GTGTTAACCT CTATCCACTT 240
   TACCAAGCTG ATTGGTTAAT AGCCCAATCG GTAAACCAGG TTTCCTGGCG ACTATAGCGG 300
   TTTGGAACCA CCTGATCCCA TCTCGAACTC AGAAGTGAAA CATACCTGCG CCAATGATAG 360
   TGTGGGGCTT CCCC 374
(2) ANGABEN ZU SEQ ID NO: 62:
   (i) SEQUENZKENNZEICHEN:
      (A) LÄNGE: 340 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella moravica
      (B) STAMM: 316-36
      (C) INDIVIDUUM/ISOLAT: 43monr
   (xi) SEQUENZBESCHREIBUNG: SEQ ID NO: 62:
   GCCTCCCTCA AGATGAGTTT TCCCATGAAG CCCGTTGAAG ACTACGACGT TGATAGGCAA 60
   GGTGTGGAAG CGCAGTAATG TGTGAAGCTA ACTTGTACTA ATTGGCTGAT TGTCTTGACC 120
   ATATAATCTG AGTTACTTCG GGTTATAGAA GTAGACGATA AAATAGAGTA GAATGTGTTA 180
   CCTCGAATCT TTACCAGGCC TTGGGCTTGT TGATTGAACN CAATCATCAA TCTGAAGGTA 240
   AACAGTTTTC CTGGCGACAA TAGCGGTTTG GAACCACCTG ATCCCATCTC GAACTCAGAA 300
   GTGAAACGAA CATGCGCCGA TGATAGTGTG AGGCTTCCTC 340
(2) ANGABEN ZU SEQ ID NO: 63:
   (i) SEQUENZKENNZEICHEN:
      (A) LÄNGE: 302 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT;
      (A) ORGANISMUS: Legionella oakridgensis
      (B) STAMM: OR-10
      (C) INDIVIDUUM/ISOLAT: 44oak
   (xi) SEQUENZBESCHREIBUNG: SEQ ID NO: 63:
   AGCCTCCCTC GAGATGAGTT TTCCCATGAA GCCCGTTGAA GACGACGACG TTGATAGGCG 60
   AGGTGTGGAA GCGTAGTAAT ACGTGAAGCT AACTCGTACT AATTGGCTGA TTGTCTTGAC 120
   CATATAACCT GAGTTGATTC AGGTTAACGC ATGCGTTTGT GTATGGCTCA ATCTTTACCA 180
   CTTGGAAGCG TAAGCTTCCA ATACCGTTTT TCCTGGCGAC CATAGCGGTT TGGAACCACC 240
   TGATACCATC CCGAACTCAG AAGTGAAACG AACGCGGGCC AATGATAGTG TGGGGCTTCC 300
   CC 302
(2) ANGABEN ZU SEQ ID NO: 64:
   (i) SEQUENZKENNZEICHEN:
      (A) LÄNGE: 323 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella rubrilucens
      (B) STAMM: WA-270A-C2
      (C) INDIVIDUUM/ISOLAT: 45rub
   (xi) SEQUENZBESCHREIBUNG: SEQ ID NO: 64:
   GCCTCCCTCA AGATGAGTTT TCCCATGAAG CCCGTTGAAG ACTACGACGT TGATAGGCGA    60
   GGTGTGGAAG CGCAGTAATG CGTGAAGCTA ACTCGTACTA ATTGGCTGAT TGTCTTGACC   120
   ATATAACCTG ATACGCTTCA GGTTATAGCA ATAACATGAA TGTGACTCTA TTYTTTACCG   180
   GCCTCATGGC CAGCGGTTAA CACCGTTGCC ACCATGACGC TTAAACCGTT TTCCTGGCGA   240
   CCATAGCAGT CTGGAACCAC CTGAATCCAT CTCGAACTCA GAAGTGAAAC AGACTCGCGC   300
   CGATGATAGT GTGAGGTTTC CTC                                           323
(2) ANGABEN ZU SEQ ID NO: 65:
   (i) SEQUENZKENNZEICHEN:
      (A) LÄNGE: 316 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella sainthelensis
      (B) STAMM: Mt.St. Helens-4
      (C) INDIVIDUUM/ISOLAT: 46saint
   (xi) SEQUENZBESCHREIBUNG: SEQ ID NO: 65:
   GCCTCCCTCA AGATGAGTTT TCCCTTGAAG CCCGTTGAAG ACTACGACGT TGATAGGCAA    60
   GGTGTGGAAG CGTAGTAATG CGTGAAGCTA ACTTGTACTA ATTGGCTGAT TGTCTTGACC   120
   ATATAATCTG AGTTACTTCA GATTGTGCTG AATATAAGAT ATAATGTTAC TCTCTTTATT   180
   TACCTGAGTA TCATGCGGCT AATGCACGAT ACTCAAAACA GTTTTCCTGG CGACCATAGC   240
   GGTTTGGTAC CACCTGATTC CATCTCGAAC TCAGAAGTGA AACGAACATG CGCCAATGAT   300
   AGTGTGAGGC TTCCTC                                                   316
(2) ANGABEN ZU SEQ ID NO: 66:
   (i) SEQUENZKENNZEICHEN:
      (A) LÄNGE: 350 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella spiritensis
      (B) STAMM: Mt. St. Helens-9
      (C) INDIVIDUUM/ISOLAT: 47spir
   (xi) SEQUENZBESCHREIBUNG: SEQ ID NO: 66:
   GCCTCCCTCA AGATGAGTTT TCCCATGAAG CCCGTTGAAG ACTACGACGT TGATAGGCGA    60
   GGTGTGGAAG CGTAGTAATG CGTGAAGCTM ACTCGTACTA ATTGGCTGAT TGTCTTGACC   120
   ATATAACCTG AATGACTTCG GGTTATTGAT ACGAAAGATA CGAAAAGAAG CAAGAACGAT   180
   TGTGTTACCG AATATCTCTT TACCAGCCTG TGGTGTGCCC TGAAGAAGAA ACAGGGTTAC   240
   GACTCAGGAT AACCGTTTTC CTGGCGATTA TAGCCGTGTG GAACCACCTG ATTCCATCTC   300
   GAACTCAGAA GTGAAACGCA CGTACGCCGA TGATAGTGTG GGGTCTCGCC              350
(2) ANGABEN ZU SEQ ID NO: 67:
   (i) SEQUENZKENNZEICHEN:
      (A) LÄNGE: 360 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella steigerwaltii
      (B) STAMM: SC-18-C9
      (C) INDIVIDUUM/ISOLAT: 48steig
   (xi) SEQUENZBESCHREIBUNG: SEQ ID NO: 67:
   GCCTCCCTCA AGATGAGTTT TCCCATGAAG CCCGTTGAAG ACTACGACGT TGATAGGCAA    60
   GGTGTGGAAG CGCAGTAATG TGTGAAGCTA ACTTGTACTA ATTGGCTGAT TGTCTTGACC   120
   ATATAATCTG AATTACTTCA GAGTGACTGA ATGTGTATAC AAGCTGTAGG TTGGCCAAGG   180
   CACAACCTAC AGAAATAAAT TGTGAACCCT TTATTTACCT AATGCATGAT TCGGGTATAA   240
   TACGCCCAAC ATCATGTAAA ACCAGTTTTC CTGGCGACCA TAGCGGTTTG GAACCACCTG   300
   ACTCCATCTC GAACTCAGAA GTGAAACAGA CCCGCGCCAA TGATAGTGTG AGGTTTCCTC   360
(2) ANGABEN ZU SEQ ID NO: 68:
   (i) SEQUENZKENNZEICHEN:
      (A) LÄNGE: 366 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (vi) URSPRÜNGLICHE HERKUNFT:
      (A) ORGANISMUS: Legionella wadsworthii
      (B) STAMM: 81-716A
      (C) INDIVIDUUM/ISOLAT: 49wad
   (xi) SEQUENZBESCHREIBUNG: SEQ ID NO: 68:
   GCCTCCCTCA AGATGAGTTT TCCCTTGAAG CCCGTTGAAG ACTACGACGT TGATAGGCAA    60
   GGTGTGGAAG CGCAGTAATG TGTGAAGCTA ACTTGTACTA ATTGGCTGAT TGTCTTGACC   120
   ATATAATCTG AGTTACTTCA GGTTAACTGA TAAGTACGTA CGAATTAGAG ATTGGGTCTA   180
   GGCCCAATCT AAAAAAAATA AAAAAATGTG AACCTTTTTA TTTAGCTATA GCATGATTAG   240
   GGTATAATAC GCCCAATTCA TGCGAAACCA GTTTTCCTGG CGACAATAGC GGCTTGGAAC   300
   CACCTGATCC CATCTCGAAC TCAGAAGTGA AACGAGCATG CGCCAATGAT AGTGTGAGGT   360
   CTCCTC                                                              366

## Beschreibung

Gegenstand der Erfindung sind Verfahren zur Amplifikation von Nukleinsäuren der Gattung Legionella und Verfahren zum gattungs- und speziesspezifischen Nachweis von Bakterien der Gattung Legionella sowie dafür geeigneter Reagenzien.

Legionellen sind aquatische, ubiquitäre gramnegative aerobe, fakultativ intrazelluläre stäbchenförmige Bakterien. Die Familie Legionellaceae besteht aus einer Gattung (Legionella) mit derzeit 48 bekannten Spezies und 51 Serogruppen. Von der wichtigsten Spezies L. pneumophila existieren 16 bekannte Serovare. Unter ihren wichtigsten Reservoiren sind Wasserleitungen, Klimaanlagen und Kühltürme. Die Infektion des Menschen erfolgt über legionellenhaltige Aerosole. Die Legionellose tritt häufig als Epidemie, z. B. über Duschköpfe aus Wannwasseranlagen, kontaminiertes Kühlwasser in Klimaanlagen, oder sporadisch auf. Eine Mensch-zu-Mensch-Übertragung ist bisher nicht beschrieben.

Legionellen-Infektionen lassen sich in zwei Gruppen unterteilen: Die Legionärskrankheit, eine akute, schwere, oft mit hohem Fieber, abdominellen Beschwerden, Kopfschmerzen, Myalgien und Verwirrtheitszuständen und anderen neurologischen Symptomen einhergehende atypische Pneumonie sowie das Pontiac-Fieber, eine mit grippeähnlichen Symptomen verlaufende selbstlimitierende Variante der Legionellose.

Die wichtigste Serogruppe von L. pneumophila, ist L. pneumophila Serogruppe 1 (L. pn. Sero. 1) als dem mit ca. 80 % häufigsten Erreger der Legionärskrankheit. Es existieren aber große regionale Unterschiede, insbesondere bei den nosokomial erworbenen Legionellosen. Im Gegensatz dazu werden bei Pontiac-Fieber überwiegend L. micdadei und andere Non-Pneumophila-Spezies als kausales Agens beschrieben.

Die Labordiagnostik der Legionellen ist schwierig. Legionellen können nur schlecht mit Fuchsin angefärbt werden, so daß man die Erreger mit der Gram-Färbung praktisch nicht darstellen kann. Legionellen wachsen nicht auf üblichen Kulturmedien, sondern nur auf Spezialnährböden und einer Atmosphäre, die 2,5 - 5 % CO₂ enthält. Die Sensitivität der Kultur ist nur ca. 20 % für L. pneumophila und ca. 5 % für andere Spezies.

Zum Nachweis von Legionellen wurden unter anderem DNS-DNS-Hybridisierungen, Pulsfeld-Elektrophorese, Ribotyping, Restriktionsfragment-Längenpolymorphismen, Fettsäure- und Ubichinon-Analysen, rRNS-Sequenzierung, RT-PCR und Southern Blot, Latex-Agglutination, Fourier-transformierte Infrarotspektroskopie, indirekte Immunfluoreszenz und Kohlenhydratutilisation (BIOLOG-System) angewandt

In Med. Microbiol. Lett. 1994; 3: 279-290 und Clin. Lab. 1994; 40: 211-216 ist die Sequenzierung von 5S-rDNS von Legionellen beschrieben.

Maiwald et al. (Mol. Cell. Probes (1994) 1, 11-14) beschreiben die Charakterisierung kontaminierender DNA in Taq-Polymerase Präparationen, wobei zwei Primer verwendet werden, die der 5S ribosomalen DNA Region von Legionella Bakterien entstammen. Durch Polymerase Kettenreaktion unter Verwendung dieser Primer wurde ein DNA-Fragment erhalten, das nicht der entsprechenden Region der LegionellaBakterien stammt, sondern von kontaminierenden bakteriellen DNA-Sequenzen in den Taq-Polymerase Präparationen.

Matsiota-Bernard et al. (J. Clin. Microbiol. (1994) 32, 1503-1505) beschreiben die Evaluierung eines kommerziell erhältlichen Amplifikationskits zur Detektion von Legionella pneumophila in klinischen Proben, wobei vier spezifische Primer aus der Legionella 5S-rRNA DNA Region verwendet werden. Für die speziesspezifische Identifizierung von Legionella pneumophila wurden drei biotinylierte Primer aus der *mip*-Region verwendet. Die amplifizierten Produkte wurden anschließend durch Hybridisierung mit spezifischen Sonden nachgewiesen. Außer Legionella pneumophila konnten noch weitere Legionella Spezies amplifiziert werden.

Oshiro et al. (Canadian. J. Microbiol. (1994) 40, 495-499) beschreiben die Modifizierung von Reagenzien in den unter D2 beschriebenen kommerziell erhältlichen Kit, um die Sensitivität des Kits zu erhöhen. Hierbei wurden die dem Kit beigefügten Primer, die bereits bei D2 gewürdigt wurden, verwendet. Es konnten ebenso verschiedene Legionella Spezies nachgewiesen werden.

Kessler et al. (J. Clin. Microbiol. (1993) 31, 3325-3328) beschreiben die Verwendung des bereits von Matsiota-Bernard et al. (siehe oben) beschriebenen, kommerziell erhältlichen Kits zur schnellen Bestimmung von Legionella Spezies in Patientenproben, wobei wiederum verschiedene Legionella Spezies unter Verwendung der bereits diskutierten Primer nachgewiesen werden konnten.

Heidrich et al. (Kli. Labor (1994) 40, 211-216) beschreiben die genetische Verwandtschaft innerhalb des Genus Legionella und DNA-Sequenz Untersuchungen an der ribosomalen DNA im Bereich der DNA-Sequenz für die 5S und 23S rRNA und im Übergangsbereich zwischen diesen beiden Genen, wobei diese Untersuchungen an 49 verschiedenen Legionellen durchgeführt wurden. Die gezeigten Ergebnisse ermöglichen die gezielte Entwicklung spezifischer Oligonukleotidsonden zur Differenzierung der Legionellen-Spezies und/ oder Unterscheidung in Pneumophila und Nicht-Pneumophila Legionellen. Lisby et al. (Eur. J. Microbiol. Infect. Dis. (1994) 13, 225-231) beschreiben einen Polymerase-Kettenreaktion-Test zur Bestimmung von Legionella Spezies in der Bronchialflüssigkeit. Der Test ermöglicht die Detektion eines 375 Basenpaare langen Fragmentes aus dem 16S rRNA Gen. Auf dem Markt ist ein Kit zum Nachweis von Legionellen in Wasserproben erhältlich. Bei einem Nachweisverfahren mit Hilfe dieses Kits wird in einem ersten Schritt ein Fragment der 5S-rDNS genusspezifisch amplifiziert. In demselben Gefäß wird ein Fragment des MIP-Gens der Spezies L. pneumophila amplifiziert. Der Kit enthält insgesamt 7 Primer zur Durchführung einer sogenannten Multiplex-PCR unter Herstellung zweier PCR-Amplifikate. Anschließend erfolgt die Detektion der Amplifikate durch Reverse Dot Blot. Das in diesem Kit realisierte Verfahren ist wegen der Notwendigkeit des Einsatzes einer großen Anzahl von Primern komplex und in der Herstellung aufwendig. Darüber hinaus ist das bekannte Verfahren im Hinblick auf die Sensitivität unbefriedigend.

Aufgabe der vorliegenden Erfindung war daher die Bereitstellung von Reagenzien, die den Nachweis von Legionellen einfacher gestalten und die weniger Komponenten beinhalten. Eine weitere Aufgabe war es, spezifischere und potentiell variablere Legionellen-nachweise zur Verfügung zu stellen.

Gegenstand der Erfindung ist daher in einem ersten Aspekt ein Verfahren zur Amplifikation von Nukleinsäuren der Gattung Legionella, in dem mit weniger als 7 Primern Nukleinsäuren aller möglichen Spezies der Gattung Legionella amplifiziert werden.

Ein weiterer Gegenstand der Erfindung ist ein Nachweisverfahren unter Verwendung des oben genannten Amplifikationsverfahrens.

Ein weiterer Gegenstand der Erfindung ist ein Verfahren zum Nachweis von Bakterien der Gattung Legionella unter Verwendung einer Nukleinsäuresonde, welche eine mindestens 15 Basen lange Sequenz aufweist, die zu mindestens 90 % homolog zu einem Teil der SEQ. ID. NO. 1 ist oder die zu mindestens 90 % komplementär zu einem Teil der SEQ. ID. NO. 1 ist.

Kern der Erfindung ist, daß Sequenzen auf dem Legionella-Genom lokalisiert wurden, die das Entwerfen von Nukleinsäuresonden erlauben, die zum Nachweis aller bislang den Legionellen zugeordneten Spezies der Gattung Legionella verwendet werden können.

Unter Amplifikation wird die Erhöhung der Konzentration der in einem Reaktionsgemisch vorhandenen Nukleinsäuren oder Teilen davon verstanden. Beispiele für solche Amplifikationsverfahren sind die Polymerase-Kettenreaktion gemäß EP-B-0 201 184, das sogenannte NASBA-Verfahren gemäß EP-A-0 329 822 sowie davon abgeleitete Verfahren.

Bei dem Verfahren der EP-A-0 201 184 wird die zu amplifizierende Nukleinsäure, die einzelsträngig vorliegt oder einzelsträngig gemacht wird, mit einem molaren Überschuß zweier Oligonukleotid-Primer unter Hybridisierungsbedingungen und in Gegenwart eines induzierenden Agens für die Polymerisation und Nukleotiden behandelt, wobei die Primer so gewählt werden, daß für jeden Strang ein zum Nukleinsäurestrang komplementäres Verlängerungsprodukt des betreffenden Primers synthetisiert wird, und daß ein Verlängerungsprodukt eines Primers, wenn es von seinem Komplement getrennt ist, als Matrize zur Synthese eines Verlängerungsprodukts des anderen Primers dienen kann, Trennen der Verlängerungsprodukte von den Matrizen, an denen sie synthetisiert wurden, und Verwendung der gebildeten Verlängerungsprodukte zur erneuten Umsetzung mit den Primern. Durch die zyklische Wiederholung der Schritte ergibt sich eine theoretisch exponentielle Vermehrung einer Nukleinsäuresequenz, die innerhalb der äußeren Hybridisierungspositionen der Primer liegt.

Bei dem Verfahren gemäß EP-A-0 329 822 wird eine Primerkonstruktion zur Erzeugung einer doppelsträngigen Nukleinsäure verwendet, in der die zu amplifizierende Nukleinsäuresequenz funktionell an einen Promotor gebunden ist. Hierzu weist einer der Primer mindestens einen Strang einer Promotorregion auf Der intermediär gebildete Transkriptionskomplex wird Bedingungen unterworfen, unter denen unter der Kontrolle des Promotors Ribonukleinsäuren unter Verwendung der zu amplifizierenden Nukleinsäure als Matrize gebildet werden. Die gebildeten Ribonukleinsäuren werden erneut zur Bildung jeweils eines neuen transkribierbaren Nukleinsäurekomplexes verwendet. Ein Vorteil dieses Systems ist, daß es isotherm geführt werden kann.

Unter Multiplex-PCR wird ein Verfahren gemäß EP-A-0 364 255 verstanden. Nach diesem Verfahren werden in einer Eintopfreaktion durch geeignete Anordnung der Hybridisierungspositionen einer Vielzahl von Primern bestimmte Fragmente von Nukleinsäuren amplifiziert, die meist unterschiedliche Länge und Position im Genom aufweisen.

Bei dem erfindungsgemäßen Verfahren handelt es sich um eine spezielle Ausführungsform der sogenannten Hybridisierungstests, die in ihren Grundzügen dem Fachmann auf dem Gebiet-der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, insbesondere in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, insbesondere 2.9.1 - 2.9.10 und Molecular Cloning, Edt. J. Sambrook et al., CSH, 1989, insbesondere 9.4.7 - 9.5.8, Bezug genommen. Dazu gehören insbesondere die bekannten Methoden zur Herstellung von markierten Nukleosidtriphosphaten, wie sie auch in EP-A-0 329 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe. Direkt nachweisbare Gruppen sind beispielsweise radioaktive (³²P), farbige oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate (rNTP oder dNTP) im allgemeinen besonders gut als Substrate von (RNS- bzw. DNS-) Polymerasen einsetzbar sind und eine anschließende Reaktion mit einem markierten Antikörper gegen das Hapten oder das hapenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Unter den Nukleinsäuren, die zur Verwendung des erfindungsgemäßen Amplifikations- und Nachweisverfahrens für Legionellen verwendet werden können, werden insbesondere genomische Nukleinsäuren verstanden. Genomische Nukleinsäuren enthalten unter anderem auch die Gene, welche für die ribosomale RNS (rRNS) codieren, sowie eine Reihe von Spacersequenzen. Diese werden nach der Größe der rRNS, für die sie codieren, benannt. In Legionellengenomen sind in dieser Reihenfolge das 16S-rRNS-Gen, das 23S-rRNS-Gen und das 5S-rRNS-Gen angeordnet. Diese Gene sind durch sogenannte Spacerregionen voneinander getrennt.

SEQ. ID. NO. 1 offenbart eine Nukleotidsequenz, die Ähnlichkeiten mit der in Spezies der Gattung Legionella enthaltenen genomischen Sequenz aufweist, die aneinander angrenzende Bereiche der 23S-rRNS, der Spacerregion zwischen 5S-rRNS- und 23S-rRNS- und des 5S-rRNS-Bereiches einschließt. Die SEQ. ID. NO. 1 schließt nur einen relativ kleinen Teil der 23S-rRNS jedoch die vollständige Spacerregion und ca. 80 % der 5S-rRNS ein.

SEQ. ID. NO. 1 stellt eine Nukleotidsequenz dar, aus welcher Teilsequenzen entnommen werden können, die gattungsspezifisch für Legionella sind.

Im Sinne der vorliegenden Erfindung soll der Begriff Nukleinsäuresonde nicht im Hinblick auf die Funktion einschränkend betrachtet werden. Die oben genannten Eigenschaften sind maßgebend. Insbesondere sollen zu den Nukleinsäuresonden auch die sogenannten Probes oder Nachweissonden gezählt werden, deren Hybridisierung mit den in der Probe vorhandenen Legionellanukleinsäuren als Maß für die Anwesenheit des Bakteriums nachgewiesen werden kann. Dazu kann die Nukleinsäuresonde bevorzugt weitere Teile enthalten, die den Nachweis oder die Amplifikation nicht wesentlich nachteilig beeinflussen. Zu solchen Molekülteilen gehören beispielsweise Markierungen zum Nachweis der Sonden, bzw. Hybriden, die diese Sonde enthalten, Gruppen, welche eine Immobilisierung der Nukleinsäuresonde an eine feste Phase erlauben, feste Phasen als solche (z. B. Beads oder Oberflächen, z. B. von Reagenz- oder Detektionsgefäßen) oder weitere, nicht mit Legionella-Sequenzen interierierende Nukleotidsequenzen. Die Ausgestaltung der Nukleinsäuresonde richtet sich daher insbesondere nach der Art, wie der Nachweis der Legionella-Nukleinsäuren geführt werden soll.

Unter einer Nukleinsäuresonde wird ein Molekül mit einer festgelegten Aufeinanderfolge von Basen verstanden, welches in der Lage ist, mit natürlichen Nukleinsäuren Hybride aufgrund von Basen-Basen-Wechselwirkungen zu bilden. Hierzu gehören natürliche und artifizielle Nukleinsäuren. Die artifiziellen Nukleinsäuren unterscheiden sich von den natürlichen Nukleinsäuren dadurch, daß sie entweder im Basenteil (z. B. durch Verwendung von Basenanaloga, z. B. 7-deaza-dGTP) oder im Grundgerüst (Ersatz der Zucker- oder/und Phosphateinheiten durch andere chemische Molekülteile, z. B. Peptide) modifiziert sind. Derartige künstliche Nukleinsäuren sind beispielsweise auch die in WO 92/20702 beschriebenen Peptidnukleinsäuren (PNA). Wesentlich für die korrekte Funktionsweise der Nukleinsäuresonden ist die spezifische Basenpaarung, wodurch die Selektivität der Sonden erreicht wird. Besonders bevorzugt besteht eine Nukleinsäuresonde aus Desoxyribonukleinsäure (DNS).

Unter einer Nukleinsäuresonde werden auch die sogenannten Primer verstanden, welche nach Hybridisierung mit Legionella-Nukleinsäuren unter Verwendung eines Enzyms verlängert werden können. Typische Verlängerungsreaktionen sind beispielsweise die Anhängung von Mononukleosidtriphosphateinheiten durch DNS-Polymerasen (z. B. E. coli DNS-Polymerase, Klenow-Fragment oder Thermus aquaticus-Polymerase) unter Verwendung der Legionella-Nukleinsäuren als Matrize. In diesem Fall wird mit den bislang bekannten Enzymen das 3'-Ende des Primers verlängert. Eine weitere Verlängerungsreaktion ist die Ligase-Reaktion, bei der die Nukleinsäuresonde mit einem weiteren Oligonukleotid enzymatisch verknüpft wird (z. B. EP-A-0 320 308). Zwischen der Polymerase- und der Ligasereaktion sind Mischformen bekannt (z. B. WO 90/01069).

Unter einer für Legionella gattungsspezifischen Nukleinsäuresonde wird eine Nukleinsäure verstanden, die mit einer Nukleinsäure hybridisiert, die eine Nukleotidsequenz-gemäß "SEQ. ID. NO. 1 bzw. deren Komplement enthält, oder die mit allen der in Tabelle 1 genannten Legionellenspezies unter denselben Stringensbedingungen hybridisiert. Besonders bevorzugt hybridisieren diese Nukleinsäuren mit allen möglichen Spezies der Gattung Legionella.

Die gattungsspezifischen Sequenzen liegen bei der Sequenz der FIG I bevorzugt im 23S- oder/und 5S-Bereich; wenngleich es bei geschickter Wahl der Hybridisierungsbedingungen auch möglich ist, gattungsspezifische Sonden auch im Spacer-Bereich zu entwerfen.

**Tab. 1**

| Erwartete Amplifikatlängen 23S-5S-Spacer-Region bei Verwendung von Primerpaar B/D | | | | | |
|---|---|---|---|---|---|
| Species/Serogroup | ATCC No. (NCTC) | Stamm | Amplikonlänge/ komplette bestimmte Länge der Sequenz | EMBL Accession Number | SEQ ID. NO. |
| L. pneumophila sero 1 | 33152 | Philadelphia-1 | 232bp/336bp | Z30431 | 26 |
| L. pneumophila sero 1 | 33153 | Knoxville-1 | 232bp/336bp | Z30432 | 27 |
| L. pneumophila sero 1 | 43108 | Benidorm 030E | 232bp/336bp | Z30433 | 28 |
| L. pneumophila sero 1 | 43112 | France 5811 | 232bp/336bp | Z30534 | 29 |
| L. pneumophila sero 1 | 43109 | OLDA | 232bp/336bp | Z30434 | 30 |
| L. pneumophila sero 1 | 43110 | Oxford 4032E | 231bp/335bp | Z30435 | 31 |
| L. pneumophila sero 1 | 43113 | Camperdown-1 | 232bp/336bp | Z30435 | 32 |
| L. pneumophila sero 2 | 33154 | Togus-1 | 232bp/336bp | Z30437 | 33 |
| L. pneumophila sero 3 | 33155 | Bloomington-2 | 232bp/336bp | Z30438 | 34 |
| L. pneumophila sero 4 | 33156 | Los Angeles-1 | 232bp/336bp | Z30439 | 35 |
| L.pneumophila sero 4 | n.a. | Portland | 232bp/336bp | Z30440 | 36 |
| L. pneumophila sero 5 | 33216 | Dallas-1E | 232bp/336bp | Z30441 | 37 |
| L. pneumophila sero 5 | (11417) | Cambridge-2 | 232bp/336bp | Z30442 | 38 |
| L. pneumophila sero 6 | 33215 | Chicago-2 | 232bp/336bp | Z30443 | 39 |
| L. pneumophila sero 7 | 33823 | Chicago-8 | 232bp/336bp | Z30444 | 40 |
| L. pneumophila sero 8 | 35096 | Concord-3 | 232bp/336bp | Z30445 | 41 |
| L. pneumophila sero 9 | 35289 | IN-23-G1-C2 | 232bp/336bp | Z30446 | 42 |
| L. pneumophila sero 10 | 43283 | Leiden-1 | 232bp/336bp | Z30447 | 43 |
| L. pneumophila sero 11 | 43130 | 797-PA-H | 232bp/336bp | Z30448 | 44 |
| L. pneumophila sero 12 | 43290 | 570-CO-H | 232bp/336bp | Z30449 | 45 |
| L. pneumophila sero 13 | 43736 | 82 A 3105 | 232bp/336bp | Z30450 | 46 |
| L. pneumophila sero 14 | 43073 | 1169-MN-H | 232bp/336bp | Z30451 | 47 |
| L. anisa | 35292 | WA-316-C3 | 267bp/371bp | Z30535 | 48 |
| L. brunensis | n.a. | n.a. | 246bp/350bp | Z30536 | 49 |
| L. cherrii | 35252 | ORW | 258bp/362bp | Z30537 | 50 |
| L. cincinnatiensis | 43753 | 72-OH-H | 213bp/317bp | Z30452 | 51 |
| L. dumoffii | 33279 | NY-23 | 255bp/359bp | Z30538 | 52 |
| L. erythra | 35303 | SE-32A-C8 | 201bp/325bp | Z30453 | 53 |
| L. feeleii sero 1 | 35072 | WO-44C | 238bp/342bp | Z30454 | 54 |
| L. feeleii sero 2 | 35849 | 691-WI-H | 238bp/342bp | Z30455 | 55 |
| L. israelensis | 43119 | Bercovier-4 | 217bp/321bp | Z30583 | 56 |
| L. jordanis | 33623 | BL-540 | 244bp/348bp | Z30539 | 57 |
| L. longbeachae sero 1 | 33462 | Long Beach-4 | 208bp/312bp | Z30456 | 58 |
| L. longbeachae sero 2 | 33484 | Tucker-1 | 208bp/312bp | Z30465 | 59 |
| L. maceachernii | 35300 | PX-1-G2-E2 | 250bp/352bp | Z30461 | 60 |
| L. micdadei | 33218 | Tatlock | 267bp/371bp | Z30460 | 61 |
| L. moravica | n.a | 316-36 | 236bp/340bp | Z30457 | 62 |
| L. oakridgensis | 33761 | OR-10 | 197bp/302bp | Z30540 | 63 |
| L. rubrilucens | 35304 | WA-270A-C2 | 219bp/324bp | Z30458 | 64 |
| L. sainthelensi | 35248 | Mt St Helens-4 | 212bp/316bp | Z30459 | 65 |
| L. spiritensis | 35249 | Mt St Helens-9 | 246bp/350bp | Z30464 | 66 |
| L. steigerwaltii | 35302 | SC-18-C9 | 256bp/360bp | Z30463 | 67 |
| L. wadsworthii | 33877 | 81-716A | 262bp/366bp | Z30462 | 68 |

Unter speziesspezifischen Nukleinsäuresonden werden Nukleinsäuren verstanden, die mit allen Serovaren einer Legionellaspezies unter denselben Stringensbedingungen hybridisieren. Solche Sonden sind mit ihrer Sequenz in Beispiel 3 wiedergegeben. Darunter befindet sich auch eine L. pneumophila-Speziessonde, die mit allen Serovaren der Spezies pneumophila, nicht jedoch mit den übrigen, in Tabelle 1 genannten, Spezies aus der Gattung Legionella hybridisiert.

Die Nukleinsäuresonden der vorliegenden Erfindung sind mindestens 15 Basen lang, besonders bevorzugt zwischen 23 und 40 Basen. Es handelt sich somit um Nukleinsäuren, welche auf einfache Weise durch chemische Synthese in sogenannten (Nukleinsäure-) Synthesizern hergestellt werden können. Sequenzen, die als gattungsspezifische Sequenz für Legionella zu gebrauchen sind, erhält man durch Aussuchen einer mindestens 15 Basen langen Sequenz aus SEQ. ID.NO. 1, wobei eine Abweichung in 1 oder 2 Basen der Gattungsspezifität in Abhängigkeit von den Hybridisierungsbedingungen in den meisten Fällen keinen Abbruch tun wird. Es ist selbstverständlich, daß die Anzahl der Abweichungen mit zunehmender Größe der Nukleinsäuresonde zunehmen kann. Erfindungsgemäß sind daher Sonden bevorzugt, die zu mindestens 90 % homolog zu einem Teil der SEQ. ID. NO. 1 sind oder die zu mindestens 90 % komplementär zu einem Teil der SEQ. ID. NO. 1 sind. Besonders bevorzugt sind Sonden, welche eine mindestens 15 Basen lange Sequenz (in direkter Aufeinanderfolge) enthalten, die streng homolog oder streng komplementär zu einem Teil der SEQ. ID. NO. 1 ist.

Die Gattungsspezifität könnte leiden, wenn die Sonde weitere Legionellaspezies-spezifische Sequenzen oder nicht-Legionella spezifische Sequenzen enthält, die eine zu spezifische bzw. zu unspezifische Hybridisierung bewirken würden. Bevorzugt sind weitere Legionella-spezifischen Sequenzen, sofern sie überhaupt vorliegen, nicht mehr als 15 Basen lang.

Desweiteren kann eine Nukleinsäuresonde im Legionella-unspezifischen Teil funktionelle Nukleotidsequenzen, wie sie beispielsweise für Promotoren oder Origins of Replication charakteristisch sind, enthalten.

Innerhalb der SEQ. ID. NO. 1 sind bestimmte Bereiche zur Auswahl von gattungsspezifischen Sequenzen insbesondere für Nachweissonden besonders bevorzugt. Besonders bevorzugte Bereiche liegen zwischen den Positionen 94 und 126, 25 und 67, 336 und 293 sowie 307 und 286. Besonders bevorzugt schließt die Sequenz der Sonde die Sequenz von Position 268 bis 296 ein.

Das erfindungsgemäße Amplifikationsverfahren kommt im Gegensatz zum Stand der Technik mit weniger als 7 Primern aus. Dies ist beispielsweise möglich dadurch, daß als Primer eine oder mehrere der erfindungsgemäßen Nukleinsäuresonden benutzt werden. Ein Amplifikationsverfahren, welches mit nur einem Primer auskommt, wird schon dadurch realisiert, daß der Primer mit der zu amplifizierenden Nukleinsäure hybridisiert, mit Mononukleosidtriphosphaten unter Einwirkung einer DNS-Polymerase verlängert wird, das Verlängerungsprodukt von der Matrizennukleinsäure getrennt und der obige Vorgang mit einem neuen Primer wiederholt wird. In jedem Zyklus wird daher pro Nukleinsäure ein Verlängerungsprodukt gebildet. Es handelt sich somit um ein lineares Amplifikationsverfahren. Exponentielle Amplifikation ist theoretisch beispielsweise dann erreichbar, wenn 2 Primer eingesetzt werden, welche die prinzipiellen Bedingungen, wie sie in der EP-A-0 201 184 beschrieben sind, erfüllen. In einer vorteilhaften Ausführungsform wird ein weiteres Primerpaar eingesetzt, welches auf dem amplifizierten Nukleinsäurefragment zwischen den Hybridisierungspositionen des ersten Primerpaares hybridisiert. Diese Ausführungsform wird gelegentlich auch als "nested PCR" bezeichnet. Darin werden insgesamt 4 verschiedene Primer eingesetzt.

Auch bei den auf Transkriptionsreaktionen beruhenden Amplifikationsverfahren werden im allgemeinen Fall 2 Nukleinsäuresonden eingesetzt, die entweder auf gegenläufigen Strängen oder auf einem Strang der zu amplifizierenden Nukleinsäure hybridisiert werden können.

Erfindungsgemäß handelt es sich für das gattungsspezifische Amplifikationsverfahren bei allen als Primer füngierenden Nukleinsäuresonden um Legionella-gattungsspezifische Sonden. In einem besonders bevorzugten Fall hybridisiert einer der Primer mit einem Strang der Legionella-Nukleinsäure in der 23S-rDNS-Region und der andere mit dem Gegenstrang in der 5S-rDNS-Region. Dadurch wird erreicht, daß das amplifizierte Teilstück der Nukleinsäuresequenz der Legionellen sowohl Teile der 23S-rDNS-Region, der 5S-rDNS-Region als auch der dazwischenliegenden Spacerregion umfaßt. Zum Verständnis muß an dieser Stelle darauf hingewiesen werden, daß sich die Amplifikate unterschiedlicher Spezies in der Sequenz unterscheiden, der zwischen den einander zugewandten Enden der ursprünglichen Primer liegt. Dabei handelt es sich eben bevorzugt um Sequenzen der Spacer-Region.

Das erfindungsgemäße gattungsspezifische Amplifikationsverfahren kann für mehrere Zwecke benutzt werden. In einer ersten Möglichkeit (z. B. im Sinne eines Screenings) kann die Summe aller in der Probe vorliegenden Spezies der Gattung Legionella nachgewiesen werden. Dies ist beispielsweise möglich, wenn die in der gattungsspezifischen Amplifikation erzeugten Amplifikate mit einer weiteren gattungsspezifischen (gewünschtenfalls nachweisbar markierten) Nukleinsäuresonde zur Hybridisierung gebracht und die gebildeten Hybride nachgewiesen werden. Dabei kann die Nukleinsäuresonde (Nachweissonde) so gewählt werden, daß sie in den Bereichen nahe den ursprünglichen Primerhybridisierungsstellen hybridisiert, jedoch wird die Aussagekraft des gattungsspezifischen Nachweises dadurch noch erhöht, daß die Nukleinsäuresonde in dem zwischen den aufeinander zu zeigenden Enden der Primer liegenden Bereich der Amplifikate hybridisiert. Besonders bevorzugt hybridisiert die gattungsspezifische Nachweissonde zwischen den Positionen 242 und 299 von SEQ. ID. NO. 1 und ist zwischen 25 und 35 Nukleotiden, besonders bevorzugt 26 bis 30 Nukleotiden lang. Mit der Veränderung der Länge der Sonde muß die Hybridisierungstemperatur entsprechend angepaßt werden. Die optimale gattungsspezifische Sonde hybridisiert zwischen den Positionen 268 und 296 und ist somit 29 Nukleotide lang.

Prinzipiell sind für den Nachweis der Amplifikate alle bekannten Nachweisformate verwendbar, beispielsweise Auftrennung nach Größe der Amplifikate (z. B. Gelelektrophorese) aber auch die Blot-Verfahren. Beispielsweise kann schon aufgrund der Größenvariabilitäten der Spacer-Region eine Differenzierung von Non-Pneumophila-Spezies im einfachen, hochauflösenden Agarosegel durchgeführt werden, was im Sinne eines Screeningverfahrens eine rasche orientierende Information liefert. Auf einen Nachweis, der aufeinem Hybridisierungsschritt mit einer Nachweissonde beruht, kann jedoch meist nicht verzichtet werden. Als besonders vorteilhaft hat sich die Verwendung des Reverse-Dot-Blot-Formats erwiesen. Hierzu wird beispielsweise eine gattungsspezifische Nukleinsäuresonde auf einer Membran immobilisiert und anschließend das Produkt der Amplifikationsreaktion auf die immobilisierten Sonden gegeben. Dazu müssen die Amplifikate gegebenenfalls vorher einzelsträngig gemacht werden. Wenn während der Amplifikation markierte Mononukleosidtriphosphate eingebaut wurden, ist der Nachweis der über die Nukleinsäuresonde immobilisierten Amplifikate nach Abwaschen nicht gebundener Nukleinsäuren auf einfache Weise möglich. Ein solches Format ist beispielsweise in der EP-A-0 237 362 beschrieben. Auf den Inhalt dieser Patentanmeldung wird hiermit vollinhaltlich Bezug genommen.

Ein Nachweis ist jedoch auch über die sogenannten Sandwich-Verfahren möglich, bei denen neben der immobilisierten Nukleinsäuresonde eine weitere, markierte gattungsspezifische Nukleinsäuresonde (Nachweissonde) eingesetzt wird, die mit den Amplifikaten an einer anderen Position hybridisiert als die Festphasen-gebundene Sonde. Dieses Verfahren ist prinzipiell in EP-B-0 079 139 beschrieben.

Das erfindungsgemäße gattungsspezifische Amplifikationsverfahren ermöglicht jedoch auch den verläßlichen und unkomplizierten Nachweis von Legionella-Spezies (z. B. für den klinischen Routinealltag) oder Gemischen von Spezies (z. B. eine Unterscheidung von L-pneumophila von non-pneumophila). Für diesen Fall kann im Anschluß an das gattungsspezifische Amplifikationsverfahren eine Hybridisierung mit einer (gewünschtenfalls markierten) speziesspezifischen Sonde (Nachweissonde) durchgeführt werden, die im amplifizierten Bereich zwischen den aufeinander zu zeigenden Enden der Primer hybridisiert. In SEQ. ID-Nos. 26 - 68 sind Sequenzen, aus welchen die speziespezifischen Sequenzen ausgewält werden können, für einzelne Spezies angegeben. Die speziesspezifischen Teile befinden sich insbesondere im Spacer-Bereich, der bevorzugt mittels der gattungspezifischen Primer amplifiziert wird. Die Lage der Amplifikate (z. B. wie in Tabelle 1 angegeben) ergibt sich aus den Hybridisierungspositionen der Primer (in Tabelle 1 ist dies das Primerpaar B/D). Besonders bevorzugte speziesspezifische Nachweissonden sind in Beispiel 3.7 angegeben.

Speziesspezifische Nachweissonden, die auf diesen Spacersequenzen beruhen, sind bevorzugt länger als 15 bp und kleiner als die gesamte Spacerregion.

Das erfindungsgemäße Verfahren ermöglicht daher den multiplen Nachweis von Legionella-Spezies unter Verwendung einer einzigen in einer vorgeschalteten gattungsspezifischen Amplifikationsreaktion hergestellten Reaktionsmischung, die Amplifikate von Teilen der in der Probe anwesenden Legionella-Spezies enthält. Ein Vorteil der Erfindung ist daher, daß sie einen einfacheren, d. h. weniger komplexen Nachweis von Bakterien der Gattung Legionella ermöglicht.

Ebenfalls Gegenstand der Erfindung ist daher ein Paar von Primern zur Amplifikation von Legionella-Nukleinsäuren, von denen einer mit einem Strang der 23S-Region und der andere mit dem Gegenstrang in der 5S-Region des Legionella-Genoms hybridisiert.

Ebenfalls Gegenstand der Erfindung ist eine doppelsträngige Legionella-spezifische Nukleinsäure, enthaltend die Spacerregion zwischen 5S-rDNS und 23S-rDNS sowie jeweils nur solche Teile der 5S-rDNS und 23S-rDNS, die im Genom direkt an die Spacerregion anschließen. Bevorzugt ist diese doppelsträngige Nukleinsäure höchstens 371 bp lang und ist das Produkt einer gattungsspezifischen Amplifikationsreaktion.

Ebenfalls Gegenstand der Erfindung ist ein Reagenzkit zum Nachweis von Legionella-Spezies enthaltend mindestens eine Legionella-gattungsspezifische Nukleinsäure und mindestens eine Legionella-speziesspezifische Sonde.

Die folgenden Beispiele sollen die Erfindung näher erläutern:

### Beispiel 1:

### Bakteriengewinnung und Anzucht

Die hier verwendeten Legionellen "Type strains" wurden auf gepuffertem Hefe-Kohle-Extraktagar (P.H. Edelstein, Laboratory Diagnosis of infections caused by legionellae, Eur. J. Clin. Microbiol. Vol. 6, 1, 4-10 (1987)) unter Zusatz von α-Ketoglutarat bei einer Inkubationstemperatur von 35°C in feuchter CO₂-Atmosphäre für mindestens 3 Tage angezüchtet. Gram-Färbungen, Mikroskopie und Inkubation von Blut-Agar-Platten zeigten kein Wachstum anderer Bakterien. Die Legionellen wurden mit 3 ml bidestilliertem Wassers von den Platten geerntet und verdünnt, mit 12.000 g zentrifügiert, der Überstand verworfen und das Zellpellet in 500 µl bidestilliertem Wasser resuspendiert. Logarithmische Verdünnungsreihen und Auszählen der Kolonien auf Agarplatten ergaben durchschnittlich 10¹⁰ KBE/ml.

### Beispiel 2:

### DNS-Aufschluß

Je ein 250 µl-Aliquot der jeweiligen Bakterienkultur wurde zur Freisetzung der DNS alkalisch lysiert (PCR: Clinical diagnostics and research; Springer Verlag Berlin/Heidelberg 1992, S. 79-80), indem 250 µl einer 200 mM NaOH-Lösung hinzugefügt, mit 100 µl Mineralöl (Sigma) überschichtet und für 20 Minuten in einem auf 95°C vorgeheizten Thermomixer inkubiert wurden. Nach Schütteln und Zentrifugation wurden 32 µl Tris-HCl (pH 5,0) zur Neutralisierung hinzugefügt, erneut geschüttelt und zentrifügiert. Kontrollproben mit bidestilliertem Wasser wurden identisch behandelt, um mögliche Kontaminationen während der Probenaufbereitung zu erkennen.

Die so gewonnenen Nukleinsäuren konnten in den Amplifikations- und Hybridisierungsexperimenten eingesetzt werden.

### Beispiel 3:

### Nachweis von Legionellen (Gattung und Spezies)

Der Nachweis von Legionellen geschah durch erfindungsgemäße gattungspezifische Amplifikation unter Einbau von mit Digoxigenin markiertem dUTP. Die markierten Amplifikate wurden anschließend mittels der Reverse Dot-Blot-Technik (Kawasaki et al.: Genetic analysis using polymerase chain reaction - amplified DNS and immobilized oligonucleotide probes: reverse dot-blot typing; in: Methods in Enzymology, Band 218, 1993) nachgewiesen.

### 1. Sonden-Herstellung (Tailing-Reaktion)

Boehringer Mannheim Terminale Transferase Kit 220-582
200 pmol Oligonukleotid
20 µl 5 x Reaktionspuffer
6 µl 25 mM CoCl₂ (Endkonzentration 1,5 mM)
8 µl 10 mM dTTP (Gesamtmenge 80 nmol)
2,4 µl Terminale Transferase (60 U)
ddH₂O ad 100 µl

Gemisch eine Stunde bei 37°C inkubieren.

### 2. Polymerasenkettenreaktion (PCR) & Digoxigenin-Markierung der PCR-Produkte

PCR 25 µl Ansätze:
3,75 µl dNTPs (1 mM Konz)
1,25 Boehringer DIG DNS Labeling mixture
2,5 µl Perkin-Elmer Puffer I
je 0,75 µl Primer (Stammlösung 10 uM)
1 µl Taq Polymerase (Perkin-Elmer) (verdünnt 1 : 10)
H₂O ad 24 µl
1 µl DNS

Thermoprofil (Primer B & D; Perkin-Elmer 9600-Thermocycler)
95° - 3 Min.
95° - 30 Sek., 54° - 25 Sek., 72° - 30 Sek.: 30 Zyklen
72° - 5 Min.
herabkühlen auf 6°

### 3. Vorbereitung der Membranen

Boehringer Mannheim positively charged Nylon membranes in dünne Streifen schneiden. Mit Bleistift beschriften und mit abgeschnittener 1 ml-Pipettenspitze Kreise in die Membran "stampfen". 1 µl (2 pmol) Sonde auftragen, 10 Minuten lufttrocknen, mit 120 ml kreuzlinken (Stratalinker®, Stratagene).

Waschen der Membran (um nicht gebundene Sonde zu entfernen):
(alle Streifen zusammen in einem 50 ml Falcon®-Röhrchen)
Waschlösung: 5 x SSPE, 0,5 % SDS
30 Minuten bei 61°
Mit bidest. H₂O kurz waschen
lufttrocknen, Streifen können nach diesem Schritt bei -20° aufbewahrt werden.

### 4. Hybridisierung

Prähybridisierung - in einzelnen, numerierten Eppis (Eppendorf-Gefäß)

| | |
|---|---|
| Lösung (je 300 ml) | 5 x SSPE |
| | 0,5 % SDS |
| | 0,5 % Dextransulfat |

30 Min. bei 61°.

### Hybridisierung

PCR-Produkt wird 10 Minuten bei 95° denaturiert und zügig nach Ende der Prähybridisierung zugegeben.
Genau eine Stunde bei 61° bei sanfter Rotation hybridisieren lassen.

Nach Abschluß der Hybridisierung werden die Membranen (im selben Eppendorf-Gefäß, mit jeweils 300 µl der folgenden Lösung) gewaschen:
2 x SSPE
0,1 % SDS

Waschschritte (unter leichtem Schütteln): zweimal 5 Min. bei Raumtemperatur und einmal 10 Min. bei 65°.

### 5. Detektion

Alle Schritte werden unter ständigem leichten Schütteln in einem 50 ml Falcon-Röhrchen durchgeführt (Puffervolumen ca. 30 ml) (Boehringer Mannheim Katalog Nr. 117504)
a) 30 Min. D1-Puffer
b) 30 Min. D2-Puffer
c) 3 µl Antikörper-Konjugatlösung mit 30 ml frischem D2-Puffer vermischen, 30 Min. inkubieren
d) 2 x 15 Min. D1-Puffer
e) kurze Inkubierung in D3-Puffer
f) Die Membranen werden mit der DNS-Seite nach oben in einer Klarsichthülle plaziert:
   45 µl NBT
   35 µl X-Phosphat-Lösung
   10 ml D3-Puffer
g) Die Farblösung zugeben, die Membranen dabei nicht bewegen. Genau 15 Min. entwickeln.
h) Stopplösung: D4-Puffer
i) lufttrocknen

| **Puffer** | | |
|---|---|---|
| 20 x SSPE | 3M NaCl | 175,3 g NaCl |
| | 0,2 M Na₂H₂PO₄ | 27,6 g Na₂H₂PO₄ |
| | 20 mM EDTA | 7,4 g EDTA |
| | pH auf 7,4 mit NaOH einstellen, aqua bidest. ad 1.000 ml | |
| | | |
| 2 x SDS | 20 g SDS | |
| | pH auf 7,2 mit HCl (ein paar Tropfen) einstellen, aqua bidest ad 1.000 ml | |
| | | |
| D1 | 100 mM Maleinsäure | |
| | 150 mN NaCl | |
| | 0,3 % (w/v) Tween 20 | |
| | pH auf 7,5 bei 20° mit NaOH einstellen | |
| | | |
| D2 | 1,0 % Blocking-Reagens (Kasein, Boehringer Kit), gelöst in D1, muß ca. eine Stunde vor Gebrauch hergestellt werden bzw. kann bei -20° aufbewahrt werden. | |
| | | |
| D3 | 100 mM TrisHCl | |
| | 100 mM NaCl | |
| | 50 mM MgCl2 | |
| | pH auf 9,5 bei 20° einstellen | |
| | | |
| D4 | 10 mM Tris HCl | |
| | 1 mM EDTA | |
| | pH auf 8,0 einstellen | |

### 6. PCR-Primer

### 7. Oligonucleotidsonden (5S-rDNS):

### 8. Nachweisverfahren

### a) Reverse dot-blot-Hybridisierung mit 4 verschiedenen Sonden

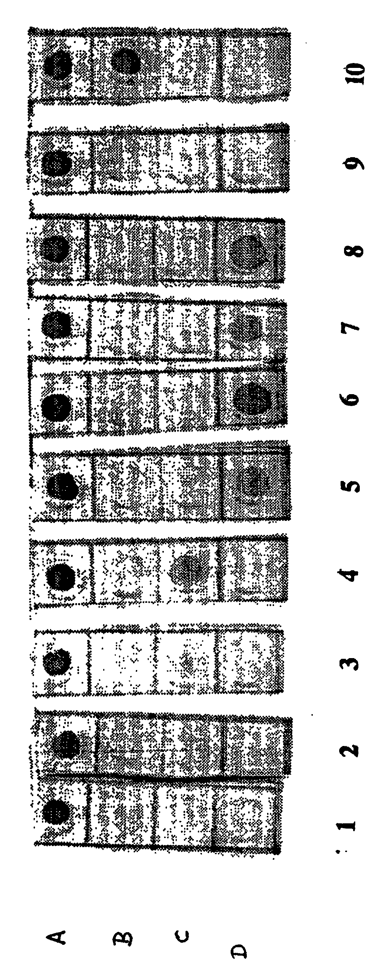

Gemäß obigem Arbeitsprotokoll wurden Nachweisverfahren unter Verwendung des gattungsspezifischen Amplifikationsverfahrens und einer gattungs- (A) bzw. 3-speziesspezifischen (B: L. pneumophila; C: L. anisa; D: L. micdadei) Sonden durchgeführt. In Figur 2 ist die Farbentwicklung an 10 Filtern gezeigt. Es ist klar erkenntlich, daß die genus-(gattungsspezifische) Sonde (A) in jedem Falle ein Nachweissignal liefert. Die L. pneumophila-spezifische Sonde (B) reagiert nur mit dem Filter, der auch den Serovar 1, Philadelphia von L. pneumophila enthält. Mit der L. anisa-spezifischen Sonde (C) ist im Filter Nr. 4 ein deutliches Signal sichtbar. Die Spezies L. micdadei ließ sich mit der L. micdadei-spezifischen Sonde (D) nachweisen. L. gormanii konnte mit der gattungsspezifischen Sonde nachgewiesen werden (Filter Nr. 9).

Die Belegung der Filter mit nachzuweisenden Nukleinsäuren war folgende:

| | | |
|---|---|---|
| 1 | L. dumofii | (2pmol probe) |
| 2 | L. anisa | (2pmol probe) |
| 3 | L. anisa | (4pmol probe) |
| 4 | L. anisa | (8pmol probe) |
| 5 | L. micdadei ATCC 33218 | (2pmol probe) |
| 6 | L. micdadei ATCC 33218 | (4pmol probe) |
| 7 | L. micdadei L 5443/90 | (2pmol probe) |
| 8 | L. micdadei L 5443/90 | (4pmol probe) |
| 9 | L. gormanii | |
| 10 | L. pneumophila sero 1 Philadelphia | |

### Amplifikation der 23S-5S-Spacer Region von Pneumophila und non-Pneumophila Spezies

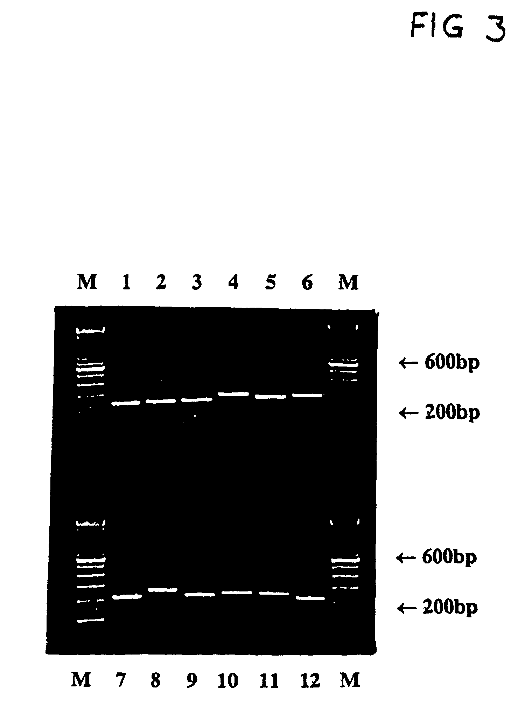

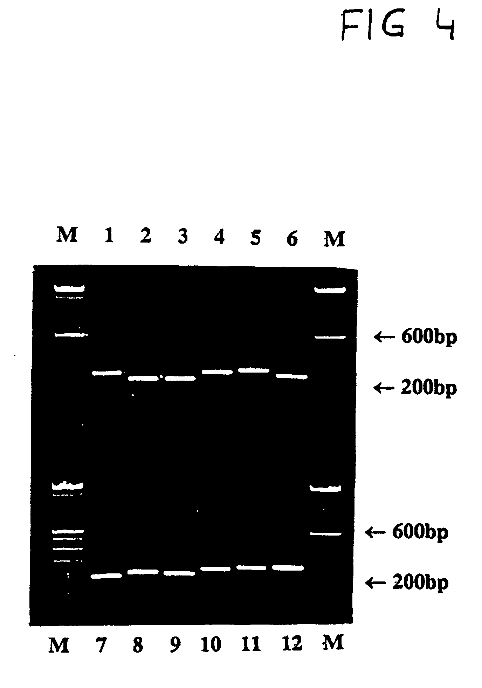

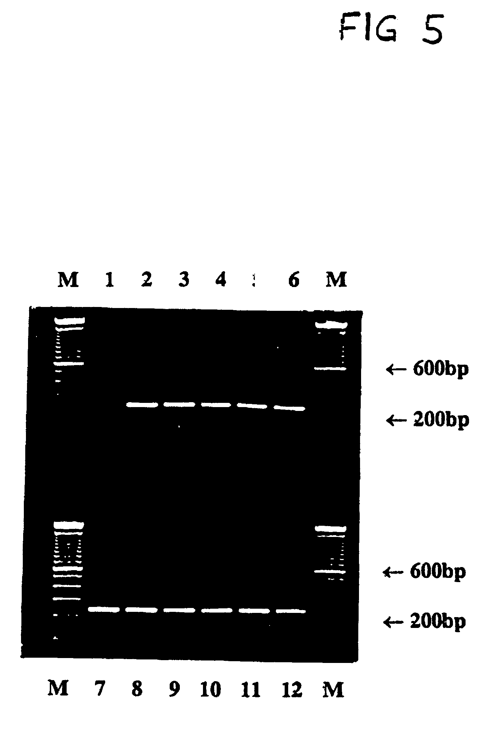

In Figur 3, 4 und 5 ist das Ergebnis der Amplifikation der 23S-5S-Spacer Region mit Hilfe der gattungsspezifischen Primer gemäß der vorliegenden Erfindung gezeigt. Es ist klar erkenntlich, daß in jedem Fall eine Amplifikation stattfindet, wobei sich in vielen Fällen die Größe der Amplifikate der einzelnen Spezies unterscheidet. Über die Größe der Amplifikate ist daher eine Unterscheidung der Spezies von Legionella nach gattungsspezifischer Amplifikation möglich.

| | | |
|---|---|---|
| 1 | L. pneumophila sero 12 570-CO-H | (FIG 3) |
| 2 | L. pneumophila sero 13 | |
| 3 | L. pneumophila sero 14 1169-MN-H | |
| 4 | L. anisa WA-316-C3 | |
| 5 | L. brunensis | |
| 6 | L. cherii ORW | |
| 7 | L. cincinattensis 72-OH-H | |
| 8 | L.dumofii NY-23 | |
| 9 | L. erythra SE-32A-C8 | |
| 10 | L. feeleii sero 1 WO-44C | |
| 11 | L. feeleii sero 2 691-WI-H | |
| 12 | L. israelensis Bercovier-4 | |
| M | 100bp DNA size marker | |

| | | |
|---|---|---|
| 1 | L. jordanis BL-540 | (FIG 4) |
| 2 | L. longeachae sero 1 Long Beach-4 | |
| 3 | L. longbeachae sero 2 Tucker-1 | |
| 4 | L. maceachernii PX-1-G2-E2 | |
| 5 | L. micdadei TATLOCK | |
| 6 | L. moravica 316-36 | |
| 7 | L. oakridgensis OR-10 | |
| 8 | L. rubrilucens WA-270-C2 | |
| 9 | L. sainthelensi Mt. St. Helens-4 | |
| 10 | L. spiritensis Mt. St. Helens-9 | |
| 11 | L. steigerwaltii SC-18-C9 | |
| 12 | L. wadsworthii 81-716A | |
| M | 100bp DNA size marker | |

| | | |
|---|---|---|
| 1 | negative control | (FIG 5) |
| 2 | L. pneumophila sero 1 Philadelphia 1 | |
| 3 | L. pneumophila sero 2 Togu-1 | |
| 4 | L. pneumophila sero 3 Bloomington-2 | |
| 5 | L. pneumophila sero 4 Los Angeles-1 | |
| 6 | L. pneumophila sero 5 Dallas-1E | |
| 7 | L. pneumophila sero 6 Chicago-2 | |
| 8 | L. pneumophila sero 7 Chicago-8 | |
| 9 | L. pneumophila sero 8 Concord-3 | |
| 10 | L. pneumophila sero 9 IN-23-G1-C2 | |
| 11 | L. pneumophila sero 19 Leiden-1 | |
| 12 | L. pneumophila sero 11 797-PA-H | |
| M | 100bp DNA size marker | |

Anstelle der in Beispiel 3 verwendeten Primer B und D können auch Primer eingesetzt werden, deren Hybridisierungspositionen auf SEQ. ID. NO. 1 abweichen. Im folgenden sind konkrete Hybridisierungspositionen (erste Basenpaarung und Länge) von weiteren Primern angegeben. Auch bezüglich der gattungsspezifischen Sonde sind im Folgenden gleichfalls-geeignete Sonden angegeben. Ebenfalls im Folgenden angegeben ist, welche Kombinationen der Primer für eine gattungsspezifische Amplifikation besonders geeignet sind.

### Beispiel 4:

### Variationsmöglichkeiten der Primen und Sonden

### Primer

### Primer B - Variationsmöglichkeiten

1) Pos. 104, 18mer, Tₘ 43,9° (Primer B aus Beispiel 3)
2) Pos. 105,21mer,Tₘ 43,0°
3) Pos. 110, 22mer, Tₘ 42,8°
4) Pos. 103, 18mer, Tₘ 43,9°
5) Pos. 101, 19mer Tₘ 42,7°
6) Pos. 100, 19mer, Tₘ 43,5°
7) Pos. 99, 20mer, Tₘ 44,2°
8) Pos. 100, 20mer, Tₘ 45,0°
9) Pos. 98, 20mer, Tₘ 43,5°
10) Pos. 94, 21mer, Tₘ 43,1°
11). Pos. 104, 19mer, Tₘ45,2°
12) Pos. 105, 23mer, Tₘ 46,1°
13) Pos. 113, 23mer, Tₘ 44,7°
14) Pos. 102, 19mer, Tₘ 46,3°
15) Pos. 100, 20mer, Tₘ 45,0°
16) Pos. 99, 21mer, Tₘ 45,6°
17) Pos. 98, 21mer, Tₘ 45,8°
18) Pos. 96, 22mer, Tₘ 45,5°
19) Pos. 105, 22mer, Tₘ 45,1°
20) Pos. 109, 23mer, Tₘ 44,0°

### Primer D - Variationsmöglichkeiten

21) Pos. 316, 20mer, Tₘ 43,7° (Primer D aus Beispiel 3)
22) Pos. 312, 19mer, Tₘ 46,0°
23) Pos. 317, 20mer, Tₘ 44,9°

### Primer A:

1) Pos. 34, 21mer, Tₘ 44,5°
2) Pos. 35, 22mer, Tₘ 45,4°
3) Pos. 37, 21mer, Tₘ 44,5°
4) Pos. 39, 20mer, Tₘ 44,6°
5) Pos. 38, 29mer, Tₘ 42,1°
6) Pos. 31, 18mer, Tₘ 43,1°
7) Pos. 29, 18mer, Tₘ 45,9°
8) Pos. 27, 18mer, Tₘ 43,2°
9) Pos. 25, 18mer, Tₘ 45,4°
10) Pos. 41, 19mer, Tₘ 43,8°

### Primer C:

11) Pos. 286, 21mer, Tₘ 44,7°
12) Pos. 286, 20mer, Tₘ 42,4°

### Variationsmöglichkeiten der 5S-Gattungssonde (nur für Primerkombination B/D)

Pos. 268, 29mer, Tₘ 61,0° (in Beispiel 3 verwendete Sonde)
Pos. 269, 29mer, Tₘ 60,7°
Pos. 270, 29mer, Tₘ 60,7°
Pos. 271, 30mer, Tₘ 61,5°
Pos. 267, 29mer, Tₘ 61,4°
Pos. 265, 27mer, Tₘ 60,6°

### Variationsmöglichkeiten der L-pheumophila Speziessonde (nur für Primerkombination B/D und A/C geeignet)

Pos. 162, 39mer, Tₘ 59,1° (in Beispiel 3 verwendete Sonde)
Pos. 160, 32mer, Tₘ 59,3°
Pos. 163, 31mer, Tₘ 60,1°
Pos. 159,33mer,Tₘ 59,4"

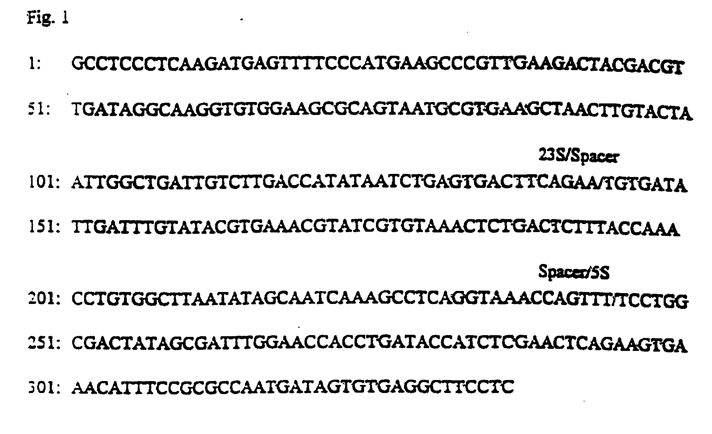

Die Positionsangaben (5'-terminale Base) beziehen sich auf die in FIG 1 abgebildete Sequenz, wobei Primer B und A komplementär zu den Teilen der Sequenz von FIG 1 sind, die bei der jeweiligen Position beginnen, und Primer D und C sequenzidentisch zu den Teilen der Sequenz von FIG 1 sind, die bei der jeweiligen Position beginnen und sich stromabwärts (von der Sequenz in FIG 1 aus gesehen) fortsetzen.

| Primerkombinationen | |
|---|---|
| Primer B/D | Primer A/C |
| 1/21 | 1/11 |
| 2/21 | 2/11 |
| 3/21 | 3/11 |
| 5/21 | 4/11 |
| 6/21 | 5/12 |
| 7/21 | 6/12 |
| 9/21 | 7/11 |
| 10/21 | 8/12 |
| 11/22 | 9/11 |
| 12/22 | 10/11 |
| 13/22 | |
| 14/22 | |
| 15/22 | |
| 16/22 | |
| 17/22 | |
| 18/22 | |
| 11/23 | |
| 4/23 | |
| 19/23 | |
| 20/23 | |
| 8/23 | |
| 17/23 | |

Alle angegebenen Primer und Nukleotidsequenzen sind DNS (Oligonukleotide im Falle der Primer und Sonden) linear, einzelsträngig.

Bevorzugt sind die erfindungsgemäßen Sonden spezifisch für Legionella, d. h., sie besitzen keine Wirkung als Primer bzw. Nachweissonden für Organismen, die nicht zur Gattung Legionella gehören. Die Primerpaare B/D und A/C wurden als nicht wirksam gegenüber Bacillus cereus, Branhamella catharrhalis, Candida albicans, Chlamydia trachomatis, Corynebacterium diphtheriae, Cryptococcus, Escherichia coli, Haemophilus influenzae, Lactobacterium, Listeria monocytogenes, Mycobacterium africanum, avium, bovis, flavescens, fortuitum, gordanae, kansasii, terrae and xenopis, Neisseria meningitidis, Nocardia, Proteus vulgaris, Pseudomonas aeruginosa, Rhodococcus, Salmonella enteriditis, Staphylococcus aureus, Streptococcus faecalis, milleri, pneumoniae and viridans and β-hemolytic Streptococcus pyogenes, Trichomonas vaginalis and Vibrio cholerae charakterisiert.

## Patentansprüche

1. Verfahren zur gattungsspezifischen Amplifikation von Nukleinsäuren der Gattung Legionella wobei mit weniger als 7 Primern Nukleinsäuren aller möglichen Spezies der Gattung Legionella amplifiziert werden, **dadurch gekennzeichnet, daß** die Primersequenzen Sequenzen enthalten, die ausgewählt sind aus Sequenzen, die zu mindestens 90 % identisch mit oder komplementär zu einem Teil der SEQ. ID. NO. 1 sind.

2. Verfahren zum Nachweis von Bakterien der Gattung Legionella in einer Probe enthaltend die Schritte
- gattungsspezifische Amplifikation von Nukleinsäuren aller in der Probe vorhandenen Spezies der Gattung Legionella, in dem mit weniger als 7 Primern Nukleinsäuren aller möglichen Spezies der Gattung Legionella amplifiziert werden und
- Nachweis einer oder mehrerer Spezies der Gattung Legionella aufgrund der Amplifikate
**dadurch gekennzeichnet, daß** die Primersequenzen Sequenzen enthalten, die ausgewählt sind aus Sequenzen, die zu mindestens 90 % identisch mit oder komplementär zu einem Teil der SEQ. ID. NO. 1 sind.

3. Verfahren gemäß Anspruch 2, **dadurch gekennzeichnet, daß** zwischen den Spezies aufgrund der unterschiedlichen Größe der Amplifikate differenziert wird.

4. Verfahren gemäß Anspruch 2, **dadurch gekennzeichnet, daß** die Amplifikate anschließend mit einer gattungsspezifischen Sonde nachgewiesen werden.

5. Verfahren gemäß Anspruch 2, **dadurch gekennzeichnet, daß** die Amplifikate anschließend mit Hilfe speziesspezifischer Sonden auf die Anwesenheit von Legionella-Spezies untersucht werden.

6. Verfahren zum Nachweis von Bakterien der Gattung Legionella unter gattungsspezifischer Amplifikation eines Teilstückes der Nukleinsäuresequenz der Bakterien, wobei das Teilstück sowohl Teile der 23S-Region und der 5S-Region, als auch die dazwischen liegende Spacerregion umfaßt **dadurch gekennzeichnet, daß** die Primersequenzen Sequenzen enthalten, die ausgewählt sind aus mindestens 15 Basen langen Sequenzen, die zumindestens 90 % identisch mit oder komplementär zu Teilsequenzen der SEQ. ID. No. 1 sind.

7. Verfahren gemäß Anspruch 6, **dadurch gekennzeichnet, daß** die Amplifikation unter Verwendung zweier Primer durchgeführt wird, von denen einer mit einem Strang in der 23S-Region und der andere mit dem Gegenstrang in der 5S-Region hybridisiert.

8. Verfahren zum Nachweis von Bakterien der Gattung Legionella unter Verwendung einer Nukleinsäuresonde, welche eine mindestens 15 Basen lange Sequenz aufweist, die zumindestens 90 % identisch zu einem Teil der 23S- oder Spacer-Region der SEQ.ID.No. 1 ist, oder die zumindestens 90 % komplementär zu einem Teil der 23S- oder Spacer-Region der SEQ. ID. No. 1 ist.

9. Verfahren gemäß Anspruch 8, **dadurch gekennzeichnet, daß** die Sonde keine weiteren Legionella-spezifischen Sequenzen aufweist, die mehr als 15 Basen lang sind.

10. Verfahren gemäß Anspruch 8, **dadurch gekennzeichnet, daß** vor Verwendung der Nukleinsäuresonde ein Amplifikationsverfahren gemäß Anspruch 8 durchgeführt wird und die Sonde mit einem Strang des amplifizierten Teilstückes hybridisieren kann.

11. Verfahren gemäß einem der Ansprüche 2 bis 10, **dadurch gekennzeichnet, daß** die Summe aller möglichen Spezies der Gattung Legionella nachgewiesen wird.

12. Nukleinsäuresonde mit einer Länge von mindestens 15 Basen zum Nachweis von Bakterien der Gattung Legionella, welche zu mindestens 90 % identisch zu einem Teil der 23S- oder Spacer-Region der SEQ.ID.No. 1 ist, oder die zu mindestens 90 % komplementär zu einem Teil der 23S- oder Spacer-Region der SEQ.ID..No.1 ist.

13. Sonde gemäß Anspruch 12 **dadurch gekennzeichnet, daß** sie gattungsspezifisch ist.

14. Sonde gemäß Anspruch 12, **dadurch gekennzeichnet, daß** der Teil von SEQ.ID.No. 1 zwischen den Positionen 94 und 126 oder 25 und 67 liegt.

15. Sonde gemäß Anspruch 12, **dadurch gekennzeichnet, daß** die Sequenz der Sonde die Sequenz von Position 94 und 126 oder 25 und 67 einschließt.

16. Paar von Primern mit einer Länge von mindestens 15 Basen zur gattungsspezifischen Amplifikation von Legionella-Nukleinsäuren von denen einer mit einem Strang in der 23S-Region und der andere mit dem Gegenstrang in der 5S-Region des Legionella-Genoms hybridisiert, **dadurch gekennzeichnet, daß** die Primersequenzen Sequenzen enthalten, die ausgewählt sind aus Sequenzen, die zu mindestens 90 % identisch mit oder komplementär zu einem Teile der SEQ. ID. NO. 1 sind.

17. Reagenzkit zur gattungsspezifischen Amplifikation und zum speziesspezifischen Nachweis von Legionella-Spezies enthaltend
- ein gattungsspezifisches Set von weniger als 7 Primern mit einer Länge von mindestens 15 Basen und
- mindestens eine Legionella-speziesspezifische Nachweis-Sonde mit einer Länge von mehr als 15 Basen
**dadurch gekennzeichnet, daß** die Primersequenzen Sequenzen enthalten, die ausgewählt sind aus Sequenzen, die zu mindestens 90 % identisch mit oder komplementär zu einem Teil der SEQ. ID. NO. 1 sind.

## Claims

1. Method for the genus-specific amplification of nucleic acids of the genus Legionella in which nucleic acids of all possible species of the genus Legionella are amplified using less than 7 primers, **characterized in that** the primer sequences contain sequences which are selected from sequences which are at least 90 % identical with or complementary to a part of SEQ.ID.NO.1.

2. Method for detecting bacteria of the genus Legionella in a sample comprising the steps
- genus-specifically amplifying nucleic acids of all species of the genus Legionella present in the sample in which nucleic acids of all possible species of the genus Legionella are amplified using less than 7 primers and
- detecting one or more species of the genus Legionella based on the amplificates,
**characterized in that** the primer sequences contain sequences which are selected from sequences which are at least 90 % identical with or complementary to a part of SEQ.ID.NO.1.

3. Method as claimed in claim 2, **characterized in that** a differentiation is made between the species based on the different sizes of the amplificates.

4. Method as claimed in claim 2, **characterized in that** the amplificates are subsequently detected using a genus-specific probe.

5. Method as claimed in claim 2, **characterized in that** the amplificates are subsequently tested for the presence of Legionella species with the aid of species-specific probes.

6. Method for detecting bacteria of the genus Legionella in which a section of the nucleic acid sequence of the bacteria is genus-specifically amplified, the said section comprising parts of the 23S region and the 5S region and also the spacer region between them, **characterized in that** the primer sequences contain sequences which are selected from sequences of at least 15 bases in length which are at least 90 % identical or complementary to partial sequences of SEQ.ID.NO. 1.

7. Method as claimed in claim 6, **characterized in that** the amplification is carried out with the aid of two primers one of which hybridizes with a strand in the 23S region and the other hybridizes with the opposite strand in the 5S region.

8. Method for detecting bacteria of the genus Legionella using a nucleic acid probe which has a sequence of at least 15 bases in length which is at least 90 % identical with a part of the 23S or spacer region of SEQ.ID.NO. 1, or is at least 90 % complementary to a part of the 23S or spacer region of SEQ.ID.NO.1.

9. Method as claimed in claim 8, **characterized in that** the probe does not contain any additional Legionella-specific sequences that are longer than 15 bases.

10. Method as claimed in claim 8, **characterized in that** prior to using the nucleic acid probe, an amplification method as claimed in claim 8 is carried out and the probe can hybridize with a strand of the amplified section.

11. Method as claimed in one of the claims 2 to 10, **characterized in that** the sum of all possible species of the genus Legionella is detected.

12. Nucleic acid probe which is at least 15 bases in length for detecting bacteria of the genus Legionella which is at least 90 % identical with a part of the 23S or spacer region of SEQ.ID.NO.1, or is at least 90 % complementary to a part of the 23S or spacer region of SEQ.ID.NO.1.

13. Probe as claimed in claim 12, **characterized in that** it is genus-specific.

14. Probe as claimed in claim 12, **characterized in that** part of SEQ.ID.NO.1 is located between positions 94 and 126 or 25 and 67.

15. Probe as claimed in claim 12, **characterized in that** the sequence of the probe includes the sequence of positions 94 and 126 or 25 and 67.

16. Pair of primers which are at least 15 bases in length for the genus-specific amplification of Legionella nucleic acids one of which hybridizes with a strand in the 23S region and the other hybridizes with the opposite strand in the 5S region of the Legionella genome, **characterized in that** the primer sequences contain sequences which are selected from sequences which are at least 90 % identical with or complementary to a part of SEQ.ID.NO.1.

17. Reagent kit for the genus-specific amplification and species-specific detection of Legionella species containing
- a genus-specific set of less than 7 primers which are at least 15 bases in length and
- at least one Legionella-species-specific detection probe which has a length of more than 15 bases
**characterized in that** the primer sequences contain sequences which are selected from sequences which are at least 90 % identical with or complementary to a part of SEQ.ID.NO.1.

## Revendications

1. Procédé pour l'amplification, spécifique à un genre, d'acides nucléiques du genre Legionella, dans lequel on procède à une amplification, avec une quantité inférieure à 7 amorces, des acides nucléiques de toutes les espèces possibles du genre Legionella, **caractérisé en ce que** les séquences d'amorçage contiennent des séquences qui ont été sélectionnées à partir de séquences qui manifestent une identité ou une complémentarité à concurrence d'au moins 90 % avec une partie de SEQ. ID. NO. 1.

2. Procédé pour le décèlement de bactéries du genre Legionella dans un échantillon, comprenant les étapes consistant à
- mettre en oeuvre une amplification spécifique à un genre, d'acides nucléiques de toutes les espèces du genre Legionella présentes dans l'échantillon, en procédant à une amplification, avec une quantité inférieure à 7 amorces, des acides nucléiques de toutes les espèces possibles du genre Legionella, et
- déceler une ou plusieurs espèces du genre Legionella en se basant sur les produits de l'amplification,
**caractérisé en ce que** les séquences d'amorçage contiennent des séquences qui ont été sélectionnées à partir de séquences qui manifestent une identité ou une complémentarité à concurrence d'au moins 90 % avec une partie de SEQ. ID. N0. 1.

3. Procédé selon la revendication 2, **caractérisé en ce qu'**on opère une différenciation entre les espèces sur base des dimensions différentes des produits de l'amplification.

4. Procédé selon la revendication 2, **caractérisé en ce qu'**on soumet ensuite les produits de l'amplification à un décèlement avec une sonde spécifique à un genre.

5. Procédé selon la revendication 2, **caractérisé en ce qu'**on analyse ensuite les produits de l'amplification à l'aide de sondes spécifiques à une espèce, quant à la présence de l'espèce Legionella.

6. Procédé pour le décèlement de bactéries du genre Legionella en soumettant à une amplification spécifique à un genre, une fraction de la séquence d'acides nucléiques des bactéries, la fraction comprenant aussi bien des parties de la région 23S et de la région 5S, que la région intercalaire disposée entre ces dernières, **caractérisé en ce que** les séquences d'amorçage contiennent des séquences qui ont été sélectionnées à partir de séquences possédant une longueur d'au moins 15 bases, qui manifestent une identité ou une complémentarité à concurrence d'au moins 90 % avec des séquences partielles de SEQ. ID. NO. 1.

7. Procédé selon la revendication 6, **caractérisé en ce qu'**on effectue l'amplification en utilisant deux amorces, l'une s'hybridant avec un brin dans la région 23S et l'autre s'hybridant avec le brin antagoniste dans la région 5S.

8. Procédé pour le décèlement de bactéries du genre Legionella en utilisant une sonde d'acides nucléiques qui présente une séquence possédant une longueur d'au moins 15 bases, qui manifeste une identité à concurrence d'au moins 90 % avec une partie de la région 23S ou de la région intercalaire de SEQ. ID. N0. 1 ou qui manifeste une complémentarité à concurrence d'au moins 90 % avec une partie de la région 23S ou de la région intercalaire de SEQ. ID. N0. 1.

9. Procédé selon la revendication 8, **caractérisé en ce que** la sonde ne présente pas d'autres séquences spécifiques à Legionella, dont la longueur est supérieure à 15 bases.

10. Procédé selon la revendication 8, **caractérisé en ce que**, avant d'utiliser la sonde d'acides nucléiques, on met en oeuvre un procédé d'amplification selon la revendication 8, et la sonde peut s'hybrider avec un brin de la fraction amplifiée.

11. Procédé selon l'une quelconque des revendications 2 à 10, **caractérisé en ce qu'**on décèle la somme de toutes les espèces possibles du genre Legionella.

12. Sonde d'acides nucléiques dont la longueur s'élève à au moins 15 bases, pour le décèlement de bactéries du genre Legionella, qui manifestent une identité à concurrence d'au moins 90 % avec une partie de la région 23S ou de la région intercalaire de SEQ. ID. N0. 1 ou qui manifestent une complémentarité à concurrence d'au moins 90 % avec une partie de la région 23S ou de la région intercalaire de SEQ. ID. N0. 1.

13. Sonde selon la revendication 12, **caractérisée en ce qu'**elle est spécifique à un genre.

14. Sonde selon la revendication 12, **caractérisée en ce que** la partie de SEQ. ID. N0. 1 se situe entre les positions 94 et 126 ou 25 et 67.

15. Sonde selon la revendication 12, **caractérisée en ce que** la séquence de la sonde englobe la séquence des positions 94 et 126 ou 25 et 67.

16. Paire d'amorces dont la longueur s'élève à au moins 15 bases, pour l'amplification, spécifique à un genre, d'acides nucléiques de Legionella, dont l'une s'hybride avec un brin dans la région 23S et l'autre s'hybride avec le brin antagoniste dans la région 5S du génome de Legionella,
**caractérisée en ce que** les séquences d'amorçage contiennent des séquences qui ont été sélectionnées à partir de séquences qui manifestent une identité ou une complémentarité à concurrence d'au moins 90 % avec une partie de SEQ. ID. N0. 1.

17. Nécessaire de réactif pour l'amplification, spécifique à un genre, et pour le décèlement, spécifique à une espèce, de l'espèce Legionella, contenant:
- un jeu, spécifique à un genre, de moins de 7 amorces possédant une longueur d'au moins 15 bases, et
- au moins une sonde de décèlement spécifique à l'espèce Legionella dont la longueur est supérieure à 15 bases,
**caractérisé en ce que** les séquences d'amorçage contiennent des séquences qui ont été sélectionnées à partir de séquences qui manifestent une identité ou une complémentarité à concurrence d'au moins 90 % avec une partie de SEQ. ID. N0. 1.
